# EUROPÄISCHE PATENTSCHRIFT

(11) **EP 3 656 334 B1**
(45) Veröffentlichungstag und Bekanntmachung des Hinweises auf die Patenterteilung: **13.05.2026**
(21) Anmeldenummer: 19209681.6
(22) Anmeldetag: 18.11.2019
(51) Int. Cl.: A61B 34/20, A61B 1/00, A61B 90/90, A61B 90/98, A61B 5/06, A61B 17/3207, A61B 17/00, A61B 90/00, A61B 1/05

(54) **ENDOSKOPVORRICHTUNG SOWIE VERFAHREN ZUM BETRIEB EINER ENDOSKOPVORRICHTUNG**
ENDOSCOPIC DEVICE AND METHOD FOR OPERATING SAME
DISPOSITIF FORMANT ENDOSCOPE ET PROCÉDÉ DE FONCTIONNEMENT D'UN DISPOSITIF FORMANT ENDOSCOPE

(30) Priorität: 20.11.2018 DE 102018129150
(43) Veröffentlichungstag der Anmeldung: 27.05.2020
(73) Patentinhaber: Karl Storz SE & Co. KG, 78532 Tuttlingen (DE)
(72) Erfinder: Fallert, Johannes, 78532 Tuttlingen (DE); Klein, Kirsten, 78532 Tuttlingen (DE)

(56) Entgegenhaltungen:
- EP-A1- 1 543 789
- WO-A1-2017/051224
- DE-A1- 102013 222 230
- US-A1- 2002 010 384
- US-A1- 2004 054 489
- US-A1- 2005 131 426
- US-A1- 2008 228 195
- US-A1- 2017 252 114

## Beschreibung

### Stand der Technik

Die Erfindung betrifft eine Endoskopvorrichtung gemäß Patentanspruch 1 sowie ein Verfahren zum Betrieb einer Endoskopvorrichtung gemäß Patentanspruch 13.

Aus der DE 10 2014 222 880 A1 ist bereits ein Adapter bekannt, mit welchem eine Endoskopkamera an einem proximalen Ende einer Endoskopbasis ankoppelbar ist. Ein solcher Adapter weist ein Spiel, insbesondere in Bezug auf eine Orientierung relativ zu weiteren Komponenten eines Endoskops auf, um eine leicht gängige Montage zu erzielen. Demnach ist ein solcher Adapter zwar für die Montage einer Endoskopkamera geeignet, jedoch nicht zu einer spielfreien Montage eines Trackermoduls, um mit diesem, insbesondere ohne stetige Nachkalibration der Lage und/oder Orientierung, ein genaues Motion Capturing zu ermöglichen.

Da auch mit anderen bekannten Adaptern die Lage und/oder Orientierung eines Trackermoduls derart nicht genau reproduzierbar ist, bedarf es demnach vor jedem Einsatz einer Durchführung eines Verfahrens einer Hand-Augen-Kalibration, welche beispielsweise in der US 6,511,418 B2 offenbart ist.

Aus der DE 2013 222 230 A1 geht zudem bereits ein chirurgisches Instrument mit einem Instrumentengriff verbundenen Instrumentenschaft und einer lösbar anordenbaren Instrumentenspitze, sowie einer Lokalisatoranordnung zum Bestimmen der Lage der Instrumentenspitze hervor. Es ist offenbart, dass ein erste Lokalisator fest am Instrumentenschaft angeordnet ist um einen Arbeitspunkt an der Instrumentenspitze des chirurgischen Instrumentes mit Hilfe eines zweiten Lokalisators in Nähe des Arbeitspunktes zu lokalisieren.

US 2002/010384 A1 offenbart eine Vorrichtung zur Kalibrierung der Linsenposition und des Sichtfelds in einem Endoskop. Die Vorrichtung umfasst Verfolgungselemente, die an festen Positionen am Schaft des Endoskops angebracht sind, eine Halterung, die ein Objekt oder Muster bereitstellt, das mit dem Endoskop betrachtet werden soll, wenn das Endoskop in die Halterung eingesetzt wird, sowie Positionselemente, die an bekannten Positionen an der Halterung angebracht sind. Ein Prozessor in der Vorrichtung ermittelt die Positionen der Verfolgungs- und Positionselemente, wobei der Endoskopschaft in der Halterungsführung aufgenommen ist, und berechnet aus den ermittelten Positionen die Koordinaten der Endoskoplinse in Bezug auf die Verfolgungselemente und das Sichtfeld der Linse. Ebenfalls offenbart wird ein Kalibrierungsverfahren, das die Vorrichtung verwendet.

Die Aufgabe der Erfindung besteht insbesondere darin, eine gattungsgemäße Endoskopvorrichtung sowie Verfahren zu deren Betrieb einer Endoskopvorrichtung mit verbesserten Eigenschaften hinsichtlich einer Genauigkeit eines Motion Capturing, insbesondere unter Berücksichtigung einer verbesserten Benutzerfreundlichkeit, bereitzustellen. Die Aufgabe wird erfindungsgemäß durch die Merkmale des Patentanspruchs 1 sowie 13 gelöst, während vorteilhafte Ausgestaltungen und Weiterbildungen der Erfindung den Unteransprüchen entnommen werden können.

### Vorteile der Erfindung

In einem Aspekt der Erfindung, welcher insbesondere einzeln oder in Kombination mit weiteren Aspekten betrachtet werden kann, wird eine Endoskopvorrichtung vorgeschlagen, mit zumindest einer Endoskopbasis, welche wenigstens einen Schaft und wenigstens eine mit dem Schaft verbundene Handhabe umfasst, mit wenigstens einer Endoskopkamera, welche zumindest teilweise in die Endoskopbasis integriert ist, und mit wenigstens einer Schnellverbinderschnittstelle, welche zu einer lösbaren Verbindung wenigstens eines Trackermoduls mit der Endoskopbasis ausgebildet ist und welche wenigstens eine Lage und/oder Orientierung des Trackermoduls relativ zur Endoskopkamera definiert, wobei wenigstens eine werkseitig bestimmte Lage- und/oder Orientierungskenngröße dieser definierten Lage und/oder Orientierung wiederabrufbar auf wenigstens einem zugeordneten Speichermedium hinterlegt ist.

Hierdurch kann vorteilhaft eine Genauigkeit einer Endoskopvorrichtung bezüglich eines Motion Capturing verbessert werden. Vorzugsweise kann erreicht werden, dass die Endoskopvorrichtung mit einer beständigen Kalibration einsetzbar ist und insbesondere eine Kalibration vor einem Einsatz durch einen Benutzer vereinfacht oder sogar vermieden werden kann. Dadurch kann insbesondere eine reproduzierbare Genauigkeit einer Anwendung der Endoskopvorrichtung erzielt werden. Zudem kann vorteilhaft eine Benutzerfreundlichkeit verbessert werden. Ganz besonders vorteilhaft kann eine Abweichung der Endoskopvorrichtung von einer definierten Lage und/oder Orientierung erkannt werden und somit eine Beschädigung der Endoskopvorrichtung und/oder eine Benutzung unvorhergesehener Komponenten der Endoskopvorrichtung ausgemacht werden. Somit kann insbesondere eine Sicherheit eines Patienten erhöht werden, da dieser vor einer Fehlbehandlung aufgrund etwaiger falsch kalibrierter, beschädigter und/oder ungeeigneter Komponenten der Endoskopvorrichtung geschützt werden kann.

Weiter kann eine Sicherheit für einen Patienten vorzugsweise durch die Verbesserung des Benutzerkomforts für einen Bediener der Endoskopvorrichtung erhöht werden, da hierdurch Ermüdungserscheinungen verringert werden. Weiterhin kann insbesondere durch eine lösbare Verbindung über die Schnellverbinderschnittstelle eine Autoklavierbarkeit der einzelnen Komponenten der Endoskopvorrichtung erzielt und somit auch eine Hygiene verbessert werden, um etwa den Hygieneanforderungen von Multi-Use-Geräten gerecht zu werden.

Unter einer "Endoskopvorrichtung" soll insbesondere ein, vorzugsweise funktionsfähiger Bestandteil, insbesondere eine Unterbaugruppe und/oder eine Konstruktions- und/oder eine Funktionskomponente eines Endoskops, insbesondere eines Videoendoskops, verstanden werden. Insbesondere kann die Endoskopvorrichtung das Endoskop vollständig umfassen. Das Endoskop ist vorzugsweise Bestandteil eines Endoskopiesystems, welches zusätzlich zum Endoskop zumindest eine Steuereinheit umfasst, welche zur Durchführung eines Verfahrens zum Betrieb der Endoskopvorrichtung des Endoskops ausgebildet ist. Ferner könnte die Endoskopvorrichtung selbst eine Steuereinheit umfassen, welche insbesondere in die Endoskopbasis, vorzugsweise in die Handhabe und besonders bevorzugt in ein von der Handhabe bereitgestelltes Gehäuse integriert sein könnte. Unter einer "Steuereinheit" soll insbesondere eine elektrische und/oder elektronische Einheit mit zumindest einer Steuerelektronik verstanden werden. Unter einer "Steuerelektronik" soll insbesondere eine Einheit mit wenigstens einer Recheneinheit, wie beispielsweise einem Prozessor, ein Logikbaustein oder dergleichen, und/oder mit wenigstens einer vorzugsweise nicht flüchtigen Speichereinheit, wie beispielsweise ein Speichermedium, vorzugsweise eine Festplatte, eine Speicherkarte oder dergleichen, verstanden werden. Insbesondere ist auf der Speichereinheit zumindest ein Betriebs-, Steuer- und/oder Regelprogramm hinterlegt, welches vorzugsweise das Verfahren zum Betrieb der Endoskopvorrichtung umfasst, und welches insbesondere dazu ausgebildet ist, von der Recheneinheit ausgeführt zu werden. Unter "ausgebildet" soll insbesondere speziell ausgelegt, ausgestattet und/oder programmiert verstanden werden. Darunter, dass ein Bauteil zu einer bestimmten Funktion ausgebildet ist, soll insbesondere verstanden werden, dass das Bauteil diese bestimmte Funktion in zumindest einem Anwendungs- und/oder Betriebszustand erfüllt und/oder ausführt.

Die Endoskopvorrichtung umfasst im vorliegenden Fall eine, insbesondere eine einzige Endoskopbasis. Alternativ könnte die Endoskopvorrichtung zumindest zwei, vorzugsweise drei und besonders bevorzugt mehrere Endoskopbasen aufweisen. Denkbar ist, dass die Endoskopbasen zumindest teilweise ineinander integriert sein können, wie dies beispielsweise bei einem Mother-Baby-Endoskop der Fall ist. Insbesondere können die mehreren Endoskopbasen zur Bereitstellung verschiedener chirurgischer Werkzeuge ausgebildet sein. Vorzugsweise ist die Endoskopbasis mehrteilig ausgebildet. Alternativ ist es denkbar, dass die Endoskopbasis einstückig ausgebildet sein kann. Unter "einstückig" soll insbesondere zumindest stoffschlüssig verbunden, beispielsweise durch einen Schweißprozess, einen Klebeprozess, einen Anspritzprozess und/oder einen anderen, dem Fachmann als sinnvoll erscheinenden Prozess, und/oder vorteilhaft in einem Stück geformt, verstanden werden, wie beispielsweise durch eine Herstellung aus einem Guss und/oder durch eine Herstellung in einem Ein- oder Mehrkomponentenspritzverfahren und vorteilhaft aus einem einzelnen Rohling.

Im vorliegenden Fall weist die Endoskopbasis eine, insbesondere eine einzige Handhabe auf. Die Handhabe weist insbesondere einen Handgriff auf. Vorzugsweise umfasst die Handhabe zumindest ein Gehäuse, in welchem weitere Komponenten der Endoskopvorrichtung anordenbar sind. Insbesondere kann die Handhabe zumindest teilweise, vorzugsweise zumindest teilweise und besonders bevorzugt vollständig aus Metall bestehen. Vorzugsweise sind der Handgriff und das Gehäuse zumindest teilweise einstückig ausgebildet. Beispielsweise ist denkbar, dass der Handgriff an das Gehäuse angespritzt sein kann. Die Handhabe besteht zumindest teilweise, vorzugsweise zumindest zu einem Großteil und besonders bevorzugt vollständig aus einem Kunststoff und zwar vorzugsweise aus einem autoklavierbaren Kunststoff, wie beispielsweise ETFE, PFA, PTFE, FEP, E-CTFE, PMP/TPX, PP, SI und/oder PVDF. Unter dem Ausdruck "zumindest zu einem Großteil" soll dabei insbesondere zumindest zu 55 %, vorteilhaft zumindest zu 65 %, vorzugsweise zumindest zu 75 %, besonders bevorzugt zumindest zu 85 % und besonders vorteilhaft zumindest zu 95 % und insbesondere aber auch vollständig verstanden werden und zwar insbesondere mit Bezug auf eine Masse und/oder ein Volumen des Bauteils. Darunter, dass "ein Bauteil und ein weiteres Bauteil zumindest teilweise einstückig verbunden/ausgebildet sind", soll insbesondere verstanden werden, dass zumindest ein Element und/oder Teil des ersten Bauteils mit zumindest einem Element und/oder Teil des zweiten Bauteils einstückig verbunden/ausgebildet ist.

Die Endoskopbasis umfasst im vorliegenden Fall einen, insbesondere einen einzigen Schaft. Unter einem "Schaft" soll insbesondere ein länglicher Teil des Endoskops verstanden werden. Unter einem "länglichen Bauteil" soll insbesondere ein Bauteil verstanden werden, dessen Haupterstreckung zumindest um einen Faktor fünf, vorzugsweise zumindest um einen Faktor zehn und besonders bevorzugt zumindest um einen Faktor zwanzig größer ist als eine Erstreckung des Bauteils senkrecht zur Haupterstreckungsrichtung, also insbesondere einem Durchmesser des Bauteils. Unter einer "Haupterstreckungsrichtung" eines Bauteils soll dabei insbesondere eine Richtung verstanden werden, welche parallel zu einer längsten Kante eines kleinsten gedachten Quaders verläuft, welcher das Bauteil gerade noch vollständig umschließt. Der Schaft weist insbesondere einen Durchmesser von zumindest 2 mm, vorzugsweise zumindest 4 mm und besonders bevorzugt zumindest 8 mm auf. Ferner weist der Schaft einen Durchmesser von höchstens 18 mm, vorzugsweise höchstens 16 mm und besonders bevorzugt von höchstens 12 mm auf. Ganz besonders bevorzugt weist der Schaft einen Durchmesser von zumindest im Wesentlichen 10 mm auf. Unter "zumindest im Wesentlichen" soll insbesondere eine maximale Abweichung eines Wertes von höchstens 10 % vorzugsweise höchstens 5% und besonders bevorzugt höchstens 2 % verstanden werden. Der Schaft ist insbesondere zumindest zu einer Anordnung wenigstens eines Beleuchtungsmoduls und/oder wenigstens eines Bilderfassungsmoduls der Endoskopvorrichtung ausgebildet. Bevorzugt ist der Schaft ausschließlich zur Anordnung des Beleuchtungsmoduls und/oder des Bilderfassungsmoduls ausgebildet. Alternativ oder zusätzlich könnte der Schaft auch zur Anordnung von Werkzeugen ausgebildet sein. Besonders bevorzugt ist der Schaft als ein Außenschaft ausgebildet. Der Schaft ist insbesondere fest mit der Handhabe verbunden. Vorzugsweise kann der Schaft zumindest teilweise einstückig mit der Handhabe ausgebildet sein. Besonders bevorzugt ist der Schaft mit der Handhabe umspritzt. Der Schaft ist vorzugsweise starr ausgebildet. Der Schaft besteht insbesondere zumindest teilweise, vorzugsweise zumindest zu einem Großteil und besonders bevorzugt vollständig aus Metall, insbesondere Stahl, vorzugsweise Edelstahl, und/oder Titan. Denkbar wäre, dass die Endoskopbasis auch zumindest zwei, vorzugsweise drei und besonders bevorzugt mehrere Schäfte aufweisen könnte. Dabei wären die Schäfte insbesondere zumindest teilweise ineinander angeordnet, wie dies beispielsweise bei einem Mother-Baby-Endoskop und/oder bei einem einen Schaft umgebenden weiteren Schaft, welcher als Spülschaft ausgebildet ist, der Fall ist. Ferner könnten die mehreren Schäfte zur Anordnung von verschiedenen Werkzeugen ausgebildet sein.

Die Endoskopkamera ist vorzugsweise zumindest zu einem Großteil und besonders bevorzugt vollständig in die Endoskopbasis, insbesondere in den Schaft der Endoskopbasis, integriert. Vorzugsweise ist die Handhabe frei von der Endoskopkamera. Insbesondere für diesen Fall ist das Endoskop als ein starres Videoendoskop ausgebildet. Andernfalls könnte das Endoskop beispielsweise auch als ein Fiberskop, Flexoskop oder dergleichen ausgebildet sein. Die Endoskopkamera umfasst insbesondere zumindest ein Bilderfassungsmodul. Das Bilderfassungsmodul weist insbesondere zumindest eine Bilderfassungsoptik auf. Die Bilderfassungsoptik umfasst insbesondere zumindest ein optisches Bauteil, wie in etwa ein Objektiv. Ferner kann die Bilderfassungsoptik weitere optische Elemente umfassen, wie beispielsweise Linsen, Prismen, Lichtwellenleiter oder dergleichen. Insbesondere weist das Bilderfassungsmodul zumindest eine Bilderfassungssensorik auf. Die Bilderfassungssensorik umfasst insbesondere zumindest einen Bildsensor, welcher vorzugsweise als ein CCD-Sensor oder CMOS-Sensor ausgebildet ist. Besonders bevorzugt ist die Endoskopkamera als eine Stereokamera ausgebildet und weist insbesondere zumindest ein weiteres Bilderfassungsmodul auf. Das weitere Bilderfassungsmodul ist insbesondere zumindest im Wesentlichen identisch zum Bilderfassungsmodul ausgebildet. Unter "zumindest im Wesentlichen identisch" soll insbesondere bis auf Herstellungs- und/oder Montagetoleranzen identisch verstanden werden. Das weitere Bilderfassungsmodul ist zumindest im Wesentlichen parallel zum Bilderfassungsmodul angeordnet. Unter "zumindest im Wesentlichen parallel" soll insbesondere eine Ausrichtung einer Richtung relativ zu einer Bezugsrichtung, insbesondere in einer Ebene, verstanden werden, wobei die Richtung und die Bezugsrichtung einen Winkel von 0°, insbesondere unter Berücksichtigung einer maximalen Abweichung von kleiner als 8°, vorteilhaft von kleiner als 5° und besonders vorteilhaft von kleiner als 2° einschließt. Das weitere Bilderfassungsmodul ist zum Bilderfassungsmodul seitlich versetzt angeordnet und zwar insbesondere zumindest im Wesentlichen senkrecht zu einer optischen Achse des Bilderfassungsmoduls, vorzugsweise einer Haupterstreckung des Schafts. Unter "zumindest im Wesentlichen senkrecht" soll hier insbesondere eine Ausrichtung einer Richtung relativ zu einer Bezugsrichtung, insbesondere in einer Ebene, verstanden werden, wobei die Richtung und die Bezugsrichtung einen Winkel von 90° insbesondere unter Berücksichtigung einer maximalen Abweichung von kleiner als 8°, vorteilhaft von kleiner als 5° und besonders vorteilhaft von kleiner als 2° einschließt.

Die Endoskopvorrichtung umfasst insbesondere zumindest einen Schnellverbinder. Unter einem "Schnellverbinder" soll insbesondere ein Verbinder verstanden werden, welcher zu einem werkzeuglosen Verbinden und zerstörungsfreien Lösen von Bauteilen ausgebildet ist, wie beispielsweise ein Bajonettverschluss, ein Magnetverschluss, einen Klettverschluss oder dergleichen. Besonders bevorzugt ist der Schnellverbinder einhändig, vorzugsweise mit einer einzigen Handbewegung betätigbar. Unter einer "Schnellverbinderschnittstelle" soll insbesondere eine Schnittstelle eines Schnellverbinders verstanden werden, welche insbesondere dazu ausgebildet ist, mit einer korrespondierenden Schnellverbinderschnittstelle zu einer Verbindung weiterer Komponenten der Endoskopvorrichtung zusammenzuwirken. Die Schnellverbinderschnittstelle ist insbesondere fest mit der Endoskopbasis, insbesondere dem Schaft verbunden. Unter "fest verbunden" soll insbesondere nicht werkzeuglos und/oder nicht zerstörungsfrei trennbar verstanden werden. Die Schnellverbinderschnittstelle weist insbesondere zumindest ein Kragenelement auf. Das Kragenelement erstreckt sich insbesondere zumindest im Wesentlichen senkrecht zu einer Haupterstreckungsrichtung der Endoskopbasis. Das Kragenelement umgreift insbesondere die Endoskopbasis, vorzugsweise den Schaft zumindest teilweise, vorzugsweise zumindest zu einem Großteil und besonders bevorzug vollständig. Unter "zumindest teilweise umgriffen" soll insbesondere zumindest zu 180° umgriffen verstanden werden. Unter "zumindest zu einem Großteil umgriffen" soll insbesondere zumindest zu 270° umgriffen verstanden werden. Unter "vollständig umgriffen" soll insbesondere zumindest zu 360° umgriffen verstanden werden. Darunter, dass "ein Bauteil ein weiteres Bauteil umgreift" soll insbesondere verstanden werden, dass bei einer umgriffenen Anordnung dieser eine Gerade existiert, welche ihren Ursprung im Bauteil hat und zunächst durch das Bauteil, dann durch das weitere Bauteil und danach sich wiederum durch das weitere Bauteil erstreckt, wobei die Gerade vorzugsweise durch ein geometrisches Zentrum des weiteren Bauteil verläuft. Vorzugsweise ist die Schnellverbinderschnittstelle zumindest teilweise einstückig mit der Endoskopbasis und zwar insbesondere dem Schaft ausgebildet. Besonders bevorzugt ist die Schnellverbinderschnittstelle einstückig mit dem Schaft ausgebildet. Beispielsweise ist der Schnellverbinder mit dem Schaft verschweißt. Die Schnellverbinderschnittstelle besteht insbesondere zumindest teilweise, vorzugsweise zumindest zu einem Großteil und besonders bevorzugt vollständig aus Metall, insbesondere Stahl, vorzugsweise Edelstahl, und/oder Titan.

Unter einem "Trackermodul" soll insbesondere ein Bauteil verstanden werden, welches zu einer Bewegungsbestimmung beim Motion Capturing ausgebildet ist. Das Trackermodul umfasst zumindest einen Marker, vorzugsweise zumindest zwei Marker, besonders bevorzugt zumindest drei Marker und ganz besonders bevorzugt mehrere Marker, welche zu einem Motion Capturing erkannt werden und vorzugsweise an verschiedenen Positionen des Trackermoduls angeordnet sind. Die Marker sind vorzugsweise als passive Marker, wie beispielsweise reflektierende Kugeln, insbesondere Glaskugeln, ausgebildet. Alternativ könnten die Marker als aktive Marker ausgebildet sein, wie beispielsweise als Leuchtioden. Besonders bevorzugt sind die Marker autoklavierbar ausgebildet. Das Trackermodul umfasst insbesondere zumindest eine Trackerebene. Unter einer "Trackerebene" soll insbesondere eine Ebene verstanden werden, welche von zumindest drei Markern des Trackermoduls aufgespannt ist. In einem verbunden Zustand erstreckt sich eine Haupterstreckungsrichtung des Trackermoduls, insbesondere der Trackerebene, zumindest im Wesentlichen parallel zu einer Haupterstreckungsrichtung der Endoskopbasis, insbesondere des Schafts. In einem verbundenen Zustand des Trackermoduls umgreift dieser zumindest teilweise, vorzugsweise zumindest zu einem Großteil und besonders bevorzugt vollständig die Endoskopbasis, insbesondere den Schaft. Das Trackermodul besteht insbesondere zumindest teilweise, vorzugsweise zumindest zu einem Großteil und besonders bevorzugt vollständig aus Metall, insbesondere Stahl, vorzugsweise Edelstahl, und/oder Titan.

Unter einer "Lage" soll insbesondere eine Position eines Bauteils bezüglich zumindest einer, vorzugsweise zumindest zwei und besonders bevorzugt zumindest drei Koordinatenachsen verstanden werden. Unter einer "Orientierung" soll insbesondere eine Verdrehung eines Bauteiles um zumindest ein, vorzugsweise zumindest zwei und besonders bevorzugt zumindest drei Koordinatenachsen verstanden werden. Unter einer "Lagekenngröße" soll insbesondere eine Kenngröße verstanden werden, anhand welcher zumindest auf eine Lage zurückgeschlossen und/oder berechnet werden kann Insbesondere kann die Lagekenngröße mit einer kartesischen Koordinate identisch sein. Die Lagekenngröße ist insbesondere ein Abstand zwischen Endoskopkamera und Trackermodul. Unter einer "Orientierungskenngröße" soll insbesondere eine Kenngröße verstanden werden, anhand welcher zumindest auf eine Orientierung zurückgeschlossen und/oder berechnet werden kann. Insbesondere kann die Lagekenngröße mit einer Polarkoordinate identisch sein. Die Orientierungskenngröße ist insbesondere ein Azimutwinkel und/oder ein Polarwinkel zwischen Endoskopkamera und Trackermodul.

Unter "werkseitig" soll insbesondere speziell ab Werk eingestellt, ausgelegt, ausgestattet und/oder programmiert verstanden werden, wobei vorzugsweise diesbezügliche Vorgänge und/oder Verfahrensschritte im Werk stattfinden und vorzugsweise nicht bei Bedienung und/oder von einem Bediener durchgeführt werden und/oder durchführbar sind. Vorzugsweise ist die im zugeordneten Speichermedium hinterlegte Lage- und/oder Orientierungskenngröße nicht von einem Bediener überschreibbar. Es ist denkbar, dass ein Bediener zusätzliche Lage- und/oder Orientierungskenngößen hinterlegen kann, welche jedoch ursprüngliche Lage- und/oder Orientierungskenngrößen nicht überschreiben, so dass die Endoskopvorrichtung und/oder das Endoskopiesystem auf diesen/diese resetbar ist.

Unter "zugeordnet" soll insbesondere unverwechselbar miteinander verknüpft verstanden werden. Das Speichermedium kann insbesondere separat von der Endoskopvorrichtung ausgebildet sein. Vorzugsweise ist das Speichermedium Teil des Endoskopiesystems. Das Speichermedium ist insbesondere über wenigstens eine Kennung, einen Schlüssel, insbesondere eine Seriennummer, des Speichermediums und/oder der Endoskopvorrichtung der Endoskopvorrichtung zugeordnet. Vorzugsweise ist das Speichermedium, insbesondere basierend auf der Kennung, dem Schlüssel, insbesondere der Seriennummer, verschlüsselt ausgebildet, so dass ein Zugriff auf dieses vorzugsweise ausschließlich bei einer passenden Zuordnung des Speichermediums zu einer vorgesehenen Endoskopvorrichtung erfolgt. Bei dem Speichermedium kann es sich insbesondere um einen Massendatenspeicher, eine Festplatte, einen Wechseldatenträger, wie beispielsweise einen USB-Massenspeicher, einen Kartenspeicher, insbesondere Flash-Speicher, ein Nur-Lesespeicher, wie beispielsweise EEPROM, eine CD und/oder DVD, handeln. Ferner könnte das Speichermedium als ein von einem Server, insbesondere einem Cloud-Server, bereitgestellter Speicherabschnitt ausgebildet sein.

Insbesondere weist die Endoskopvorrichtung zumindest ein elektrisches und/oder elektronisches Schnittstellenmodul auf. Das elektrische und/oder elektronische Schnittstellenmodul ist zu einer elektrischen und/oder elektronischen Verbindung der Endoskopvorrichtung mit weiteren Komponenten, vorzugsweise weiteren Komponenten des Endoskopiesystems, wie in etwa der Steuereinheit und/oder dem Speichermedium, ausgebildet. Das elektrische und/oder elektronische Schnittstellenmodul weist vorzugsweise einen kabelgebundenen Anschluss auf, wie beispielsweise einen USB-, BUS- und/oder LAN-Anschluss. Alternativ oder zusätzlich kann das elektrische und/oder elektronische Schnittstellenmodul zumindest einen kabellosen Anschluss umfassen, wie in etwa Wi-Fi, Bluetooth, RFID oder dergleichen. Das elektrische und/oder elektronische Schnittstellenmodul ist insbesondere zumindest teilweise, vorzugsweise zumindest zu einem Großteil und besonders bevorzugt vollständig in die Endoskopbasis, insbesondere die Handhabe und vorzugsweise in das von der Handhabe bereitgestellte Gehäuse integriert.

Das elektrische und/oder elektronische Schnittstellenmodul umfasst insbesondere zumindest eine Energieschnittstelle. Die Energieschnittstelle ist insbesondere zur Versorgung von weiteren Komponenten der Endoskopvorrichtung mit elektrischer Energie ausgebildet. Bei der Energieschnittstelle kann es sich beispielsweise um einen Netzanschluss, ein Netzteil und/oder um ein Batteriefach handeln. Vorzugsweise ist die Energieschnittstelle kabelgebunden, in etwa von dem kabelgebundenen Anschluss des elektrischen und/oder elektronischen Schnittstellenmoduls, ausgebildet.

Das elektrische und/oder elektronische Schnittstellenmodul umfasst insbesondere zumindest eine Datenschnittstelle. Die Datenschnittstelle ist insbesondere zum Austausch von Daten, insbesondere der Lage- und/oder der Orientierungskenngröße, mit weiteren Komponenten der Endoskopvorrichtung und/oder des Endoskopiesystems, insbesondere der Steuereinheit und/oder dem Speichermedium ausgebildet. Die Datenschnittstelle kann kabelgebunden, in etwa von dem kabelgebundenen Anschluss des elektrischen und/oder elektronischen Schnittstellenmoduls ausgebildet sein. Vorzugsweise ist die Datenschnittstelle kabellos, in etwa von dem kabellosen Anschluss des elektrischen und/oder elektronischen Schnittstellenmoduls, ausgebildet.

Weiterhin wird vorgeschlagen, dass auf dem Speichermedium zumindest eine Kamerakenngröße der Endoskopkamera hinterlegt ist. Hierdurch kann vorteilhaft eine Genauigkeit einer Hand-Augen-Kalibration verbessert werden. Ferner kann eine Zuordnung verbauter Komponenten verbessert werden. Insbesondere können zumindest zwei vorzugsweise zumindest drei und besonders bevorzugt mehrere Kamerakenngrößen der Endoskopkamera auf dem Speichermedium hinterlegt sein. Unter einer "Kamerakenngröße" soll insbesondere eine Kenngröße verstanden werden, welche für eine spezifische Endoskopkamera charakteristisch ist und welche vorzugsweise bei einem Kalibriervorgang und/oder einer Reevaluationsvorgang einer Hand-Auge-Kalibration zu einer Bestimmung einer Orientierung und/oder Lager der Endoskopkamera relativ zum Trackermodul beiträgt und/oder zu berücksichtigen ist. Vorzugsweise handelt es sich bei der Kamerakenngröße um eine Fokuslänge, eine Verzeichnung, eine Bildmitte, eine Blickrichtung, einen Blickwinkel der Endoskopkamera.

Denkbar ist, dass das Speichermedium zumindest Teil des Endoskopiesystems, und bevorzugt Teil des Endoskops, der Endoskopvorrichtung und/oder der Steuereinheit ist. Insbesondere um Benutzerfreundlichkeit weiter zu verbessern und eine Zuordnung zu vereinfachen, wird jedoch in einer Ausgestaltung der Erfindung vorgeschlagen, dass die Endoskopvorrichtung das Speichermedium, welches zumindest teilweise, vorzugsweise zumindest zu einem Großteil und besonders bevorzugt vollständig, in die Endoskopbasis integriert ist, umfasst. Bevorzugt ist das Speichermedium zumindest teilweise, vorzugsweise zumindest zu einem Großteil und besonders bevorzugt vollständig, in die Handhabe und besonders bevorzugt in das von der Handhabe bereitgestellte Gehäuse integriert.

Ferner wird vorgeschlagen, dass die Endoskopkamera zumindest teilweise, vorzugsweise zumindest zu einem Großteil und besonders bevorzugt vollständig, an einem distalen Endabschnitt des Schafts angeordnet ist. Aufgrund der festen Anordnung der Endoskopkamera relativ zur Schnellschnittstelle kann insbesondere eine Genauigkeit der Lage- und/oder Orientierungskenngröße weiter verbessert werden. Weiter vorteilhaft kann eine kompakte Anordnung erzielt werden. Ferner können auf weitere Bauteile, welche zu einer Abbildung des Bildes, wie beispielsweise zusätzliche Optiken und/oder Lichtwellenleiter, verzichtet werden. Unter einem "distalen Endabschnitt" eines Bauteils soll insbesondere ein Endabschnitt verstanden werden, welcher sich ausgehend von einem distalen Ende des Bauteils in proximaler Richtung erstreckt. Unter einem "proximalen Endabschnitt" eines Bauteils soll insbesondere ein Endabschnitt verstanden werden, welcher sich ausgehend von einem proximalen Ende des Bauteils in distaler Richtung erstreckt. Unter einem "Endabschnitt" eines Bauteils soll insbesondere ein Abschnitt verstanden werden, welcher sich ausgehend von einem Ende des Bauteils zur Mitte des Bauteils hin um höchstens 10 cm, vorzugsweise um höchstens 5 cm und besonders bevorzugt um höchstens 3 cm, erstreckt. Unter "proximal" soll insbesondere bei einer Bedienung einem Patienten abgewandt und/oder einem Bediener zugewandt verstanden werden. Unter "distal" soll insbesondere bei einer Bedienung einem Patienten zugewandt und/oder einem Bediener abgewandt verstanden werden. Der distale Endabschnitt liegt insbesondere einem proximalen Endabschnitt des Schafts gegenüber. Die Endoskopkamera ist insbesondere zumindest teilweise, vorzugsweise zumindest zu einem Großteil und besonders bevorzugt vollständig an dem distalen Ende des Schafts angeordnet. Derart kann das Endoskop in etwa als ein Videoendoskop ausgebildet sein.

Weiterhin wird vorgeschlagen, dass die Schnellverbinderschnittstelle wenigstens einen stirnseitigen Anschlag umfasst, welcher dazu ausgebildet ist, die Lage des Trackermoduls relativ zur Endoskopkamera festzulegen. Es kann eine Genauigkeit der Lage weiter verbessert werden. Insbesondere kann ein Benutzerkomfort erhöht werden, da ein Bediener bei einer Verbindung nicht auf eine richtige Positionierung der Lage der Komponenten achten muss und diese auch nicht falsch anordnen kann. Der Anschlag ist zumindest teilweise von dem Kragenelement der Schnellverbinderschnittstelle ausgebildet und zwar insbesondere von einer Stirn des Kragenelements.

Ferner wird vorgeschlagen, dass die Schnellverbinderschnittstelle zumindest ein Poka-Yoke-Element umfasst, welches dazu ausgebildet ist, die Orientierung des Trackermoduls relativ zur Endoskopkamera festzulegen. Es kann eine Genauigkeit der Orientierung weiter verbessert werden. Insbesondere kann ein Benutzerkomfort erhöht werden, da ein Bediener bei einer Verbindung nicht auf eine richtige Positionierung der Orientierung der Komponenten achten muss und diese auch nicht falsch anordnen kann. Das Poka-Yoke-Element ist insbesondere als eine Nut und/oder ein Pin ausgebildet. Das Poka-Yoke-Element ist insbesondere zumindest teilweise von dem Kragenelement ausgebildet und zwar insbesondere von einem, vorzugsweise in/auf einem Mantel des Kragenelements, in das Kragenelement angeordneten Nut und/oder Pin das Poka-Yoke-Element.

Ferner wird vorgeschlagen, dass die Schnellverbinderschnittstelle zumindest teilweise, vorzugsweise zumindest zu einem Großteil und besonders bevorzugt vollständig, an einem proximalen Endabschnitt des Schafts angeordnet ist. Aufgrund der festen Anordnung der Schnellverbinderschnittstelle relativ zur Endoskopkamera kann insbesondere eine Genauigkeit der Lage- und/oder Orientierungskenngröße weiter verbessert werden. Weiter vorteilhaft kann eine kompakte Anordnung erzielt werden.

Das Trackermodul könnte Teil des Endoskopiesystems sein. Insbesondere um Benutzerfreundlichkeit weiter zu verbessern und eine Zuordnung zu vereinfachen, wird jedoch in einer Ausgestaltung der Erfindung vorgeschlagen, wonach die Endoskopvorrichtung das Trackermodul, welches wenigstens eine Trackerhalterung und wenigstens einen an der Trackerhalterung angeordneten Tracker umfasst, aufweist. Die Trackerhalterung weist eine Form eines Hohlzylinders auf. In einem verbundenen Zustand des Trackermoduls umgreift die Trackerhalterung zumindest teilweise vorzugsweise zumindest zu einem Großteil und besonders bevorzugt vollständig die Endoskopbasis, insbesondere den Schaft. Der Tracker umfasst insbesondere die Marker des Trackermoduls. Ferner weist der Tracker vorzugsweise wenigstens eine Markerhalterung auf, an welcher die Marker angeordnet sind. Die Markerhalterung weist insbesondere eine Haupterstreckung auf, welche sich höchstens zu einem Teil zumindest im Wesentlichen parallel entlang des Schafts erstreckt. Ferner erstreckt sich die Haupterstreckung höchstens zu einem Teil zumindest im Wesentlichen parallel zur Haupterstreckung der Handhabe. Eine Projektion der Markerhalterung senkrecht zur proximal und/oder distalen Richtung bedeckt den Schaft zu höchstens 20 % seiner Haupterstreckung. Die Markerhalterung ist mit der Trackerhalterung verbunden. Vorzugsweise ist die Trackerhalterung mit der Markerhalterung einstückig ausgebildet. Beispielsweise kann die Markerhalterung mit der Trackerhalterung verschweißt sein. Die Trackerhalterung und/oder der Tracker, insbesondere die Markerhalterung besteht/bestehen insbesondere zumindest teilweise, vorzugsweise zumindest zu einem Großteil und besonders bevorzugt vollständig aus Metall, insbesondere Stahl, vorzugsweise Edelstahl, und/oder Titan. Vorzugsweise bildet/bilden zumindest eine Markerhalterung und zumindest drei Marker, welche an der Markerhalterung angeordnet sind einen Tracker aus.

Zudem wird vorgeschlagen, dass das Trackermodul wenigstens zwei verschiedene Trackerebenen aufweist. Es kann vorteilhaft eine Genauigkeit weiter verbessert werden, da beispielsweise gleichzeitig verschiedene Trackerebenen erfasst und/oder kalibrierbar sind. Vorteilhaft kann eine Benutzerfreundlichkeit weiter verbessert werden, indem ein Tracking möglich bleibt, selbst wenn eine der Trackerebenen hinter den Endoskopbasis und/oder einer Hand des Bedieners verdeckt ist. Unter "verschiedenen Trackerebenen" soll insbesondere Trackerebenen verstanden werden, welche zueinander winklig angeordnet sind und welche vorzugsweise von verschiedenen Markern aufgespannt werden. Vorzugsweise umfasst das Trackermodul für jede Trackerebene eine separate Markerhalterung. Die Markerhalterungen können dabei jedoch vorteilhaft miteinander verbunden sein. Ferner kann auch jede der Markerhalterung separat mit der Trackerhalterung verbunden sein. Die Trackerebenen sind insbesondere um einen Winkel zueinander versetzt um die Endoskopbasis herum angeordnet. Ferner ist denkbar, dass das Trackermodul mehrere verschiedene Tracker umfasst, welche jeweils eine Trackerebene aufweisen.

Es wird ferner vorgeschlagen, dass das Trackermodul wenigstens eine weitere Schnellverbinderschnittstelle umfasst, welche korrespondierend zur Schnellverbinderschnittstelle ausgebildet ist und mit dieser bei einer Verbindung des Trackermoduls mit der Endoskopbasis zusammenwirkt. Es kann vorteilhaft eine Genauigkeit verbessert werden, da insbesondere eine Verbindung des Trackermoduls mit der Endoskopbasis über die Schnellverbinderschnittstellen vorgegeben ist. Ferner kann eine Benutzerfreundlichkeit weiter verbessert werden. Darunter dass "ein Bauteil korrespondierend zu einem weiteren Bauteil ausgebildet ist" soll insbesondre verstanden werden, dass die Bauteile zueinander korrespondierende Formen aufweisen, wie dies beispielsweise bei ineinander passenden Schlüssel und Schlüsselloch der Fall ist. Insbesondere weisen die Schnellverbinderschnittstellen stirnseitig korrespondierende Verbindungsformen auf. Die Verbindungsform weist insbesondere eine zumindest zweifache Drehsymmetrie auf. Die Verbindungsform weist jedoch insbesondere keine Rotationssymmetrie auf. Die Verbindungsform weist in einer Draufsicht eine Formgebung eines Vierecks mit an zumindest zwei vorzugsweise gegenüberliegenden gerundeten Seiten auf.

Ferner wird vorgeschlagen, dass in wenigstens einer Anordnung der Schnellverbinderschnittstellen zueinander, die Schnellverbinderschnittstelle und die weitere Schnellverbinderschnittstelle stirnseitig ineinander einführbar sind und in wenigstens einer weiteren Anordnung stirnseitig aneinander anliegen. Es kann vorteilhaft eine Benutzerfreundlichkeit verbessert werden, indem eine Montage vereinfacht wird. In der ersten Anordnung sind insbesondere die Verbindungsformen der Schnellverbinderschnittstellen zueinander kongruent. In der zweiten Anordnung sind die Verbindungsformen zueinander verschränkt. Vorzugsweise sind in der Anordnung die Schnellverbinderschnittstellen zueinander gleich ausgerichtet und in der weitern Anordnung zueinander um einen Winkel verdreht, welcher zumindest im Wesentlichen ein Winkel ist, welcher vorzugsweise einem Kreiswinkel von 180° geteilt durch die Drehsymmetrie der Verbindungsform entspricht. Unter "zumindest im Wesentlichen" soll hier unter Berücksichtigung einer maximalen Abweichung eines Werts von einem Referenzwert von höchstens 15 %, vorzugsweise höchstens 10 % und besonders bevorzug von höchstens 5 % verstanden werden.

Ferner wird vorgeschlagen, dass die Endoskopvorrichtung zumindest einen Schnellspanner umfasst, welcher die Schnellverbinderschnittstellen bei einer Verbindung des Trackermoduls mit der Endoskopbasis durch Kraftbeaufschlagung miteinander verspannt. Es kann vorteilhaft eine besonders sichere Verbindung der Komponenten positionsgenau erfolgen. Unter einem "Schnellspanner" soll insbesondere ein Spanner verstanden werden, welcher zu einem werkzeuglosen Verspannen und/oder Verklemmen und zerstörungsfreien lösen einer Verspannung und/oder Verklemmung von Bauteilen ausgebildet ist. Der Schnellspanner weist zumindest eine erste Position, in welcher dieser die Schnellverbinderschnittstellen löst und eine zweiten Position, in welcher dieser die Schnellverbinderschnittstellen, miteinander verbindet, auf. Der Schnellspanner umfasst zumindest einen Spannring. Der Spannring ist drehbar gelagert und zwar insbesondere um den Schaft. Vorzugsweise ist der Spannring durch eine Drehbewegung von der ersten Position des Schnellspanners in die zweite Position des Schnellspanners überführbar und umgekehrt. Der Schnellspanner weist insbesondere zumindest ein Betätigungselement auf, welches zu einer Betätigung des Schnellspanners und zwar insbesondere des Spannrings ausgebildet ist, und welches insbesondere an dem Spannring vorzugsweise fest angeordnet ist. Das Betätigungselement ist bevorzugt stiftförmig ausgebildet. Der Schnellspanner weist insbesondere zumindest ein Klemm- und/oder Spannelement auf, mittels welchem der Schnellspanner die Schnellverbinderschnittstellen in der zweiten Position miteinander verklemmt und/oder verspannt werden. Das Klemm- und/oder Spannelement ist vorzugsweise als ein Gewinde, welches zumindest ein Sechzehntel, vorzugsweise zumindest ein Achtel und besonders bevorzugt zumindest ein Viertel Gewindegang und/oder höchstens einen, vorzugsweise höchstens Dreiviertel und besonders bevorzugt höchstens einen Halben Gewindegang umfasst, ausgebildet. Alternativ oder zusätzlich kann der Schnellspanner ein Klemm- und/oder Spannelement aufweisen, welches beispielsweise als ein elastisches Element ausgebildet ist, wie in etwa eine Feder oder ein elastisches Gummi. Der Schnellspanner weist ferner insbesondere ein weiteres Klemm- und/oder Spannelement auf, welches korrespondierend zum Klemm- und/oder Spannelement ausgebildet ist. Das weitere Klemm- und/oder Spannelement ist vorzugsweise von dem Klemm- und/oder Spannelement geführt. Vorzugsweise ist das weiter Klemm- und/oder Spannelement als ein Zapfen, Bolzen oder dergleichen ausgebildet. Das weitere Klemm- und/oder Spannelement ist insbesondere an dem Spannring vorzugsweise fest angeordnet. Besonders bevorzugt ist das weitere Klemm- und/oder Spannelement einstückig mit dem Betätigungselement des Schnellspanners ausgebildet.

Es wird zudem vorgeschlagen, dass der Schnellspanner zumindest teilweise einstückig mit dem Trackermodul ausgebildet ist. Hierdurch kann eine Bedienerfreundlichkeit verbessert werden, da insbesondere auf zusätzliche Einzelteile verzichtet und somit eine Komplexität verringert werden kann. Ferner kann eine durch weitere Einzelteile verursacht erhöht auftretende Fehlertoleranz vermieden werden, wodurch eine Genauigkeit verbessert werden kann. Das Klemm- und/oder Spannelement ist vorzugsweise von einer Ausnehmung der Trackerhalterung ausgebildet. Ferner ist insbesondere der Schnellverbinder zumindest teilweise einstückig mit dem Schnellspanner ausgebildet. Insbesondere ist die korrespondierende Schnellverbinderschnittstelle des Schnellverbinders einstückig mit dem Spannring des Schnellspanners ausgebildet.

In einem weiteren Aspekt der Erfindung, welcher insbesondere einzeln oder in Kombination mit weiteren Aspekten der Erfindung betrachtet werden kann, wird ein Verfahren zum Betrieb einer Endoskopvorrichtung vorgeschlagen, bei welchem in wenigstens einem Kalibriervorgang wenigstens eine werkseitig bestimmte Lage- und/oder Orientierungskenngröße einer Lage und/oder Orientierung wenigstens eines Trackermoduls relativ zu wenigstens einer Endoskopkamera, zumindest teilweise in eine Endoskopbasis, die wenigstens einen Schaft und wenigstens eine mit dem Schaft verbundene Handhabe umfasst, integriert ist, wiederabrufbar auf wenigstens einem zugeordneten Speichermedium hinterlegt wird, wobei diese Lage und/oder Orientierung von wenigstens einer Schnellverbinderschnittstelle, welche eine lösbare Verbindung des Trackermoduls mit der Endoskopbasis herstellt, definiert wird.

Hierdurch kann vorteilhaft eine Genauigkeit einer Endoskopvorrichtung bezüglich eines Motion Capturing verbessert werden. Vorzugsweise kann erreicht werden, dass die Endoskopvorrichtung beständig einsetzbar ist und insbesondere eine Kalibration vor einem Einsatz durch einen Benutzer vereinfacht oder sogar vermieden werden kann.

Unter einem "Verfahren zum Betrieb einer Endoskopvorrichtung" soll insbesondere ein, vorzugsweise funktionsfähiger, Bestandteil, insbesondere eine Unterbaugruppe und/oder eine Konstruktions- und/oder eine Funktionskomponente, eines Betriebsprogramms der Endoskopvorrichtung verstanden werden. Unter einem Vorgang eines Verfahrens soll insbesondere ein Teil eines Verfahrensschritts verstanden werden, welcher zumindest einen, vorzugsweise zumindest einen und besonders bevorzugt mehrere Verfahrensschritte umfasst. Insbesondere kann die Verfahren zum Betrieb einer Endoskopvorrichtung das Betriebsprogramm vollständig umfassen. Das Verfahren ist insbesondere zumindest mittels der Steuereinheit des Endoskopiesystems ausführbar. Das Verfahren ist insbesondere frei von einer durch einen Bediener vor oder bei einem Betrieb durchgeführten Hand-Auge-Kalibration. Weist die Endoskopvorrichtung insbesondere verschiedene Trackerebenen auf, wird das Verfahren zum Betrieb des Endoskopiesystems zumindest teilweise, vorzugsweise zumindest zu einem Großteil und besonders bevorzugt vollständig für die verschiedenen Trackerebenen wiederholt. Ferner wird in dem Kalibriervorgang insbesondere zumindest eine Kamerakenngröße auf dem Speichermedium wiederabrufbar hinterlegt.

Ferner wird vorgeschlagen, dass in dem Kalibriervorgang mittels einer werkseitigen Hand-Augen-Kalibration die Lage- und/oder Orientierungskenngröße bestimmt wird. Es kann vorteilhaft eine Bedienerfreundlichkeit erleichtert werden, da so eine Durchführung einer Kalibration vor jedem Einsatz durch einen Bediener vermieden werden kann. Insbesondere kann eine Sicherheit weiter verbessert werden.

Ferner wird vorgeschlagen, dass das Verfahren wenigstens einen Testvorgang umfasst, bei welchem ein Abgleich einer vorliegenden Komponentenzuordnung wenigstens des Trackermoduls und der Endoskopbasis mit einer auf dem Speichermedium hinterlegten vorgesehenen Komponentenzuordnung durchgeführt wird. Es kann vorteilhaft eine Bedienerfreundlichkeit weiter verbessert werden, da vermieden werden kann, dass nicht aufeinander abgestimmte und/oder kalibrierte Komponenten, wie beispielsweise das Trackermodul und die Endoskopbasis, miteinander kombiniert werden. Dadurch kann insbesondere eine Sicherheit und Genauigkeit weiter erhöht werden. Es wird insbesondere eine unvorhergesehene Komponentenzuordnung erkannt, wenn zumindest das Trackermodul oder die Endoskopbasis von der vorgesehenen Komponentenzuordnung abweicht. Vorzugsweise wird einem Bediener eine Warnung, beispielsweise über die Anzeige, ausgegeben, wenn eine unvorhergesehene Komponentenzuordnung vorliegt. Besonders bevorzugt blockiert wird bei einer unvorhergesehenen Komponentenzuordnung ein Betrieb der Endoskopvorrichtung blockiert und zwar insbesondere durch die Steuereinheit. Die Komponentenzuordnung erfolgt insbesondere durch Abgleich einer Kennung der jeweiligen Komponenten, wie beispielsweise einer Seriennummer, welche auf den Komponenten abgebildet ist und/oder in dem Speichermedium hinterlegt sein kann. Ferner könnte eine Komponentenzuordnung mittels abrufen und/oder Abgleichen von RFID-Chips mit welchen die Komponenten ausgestattet sind erfolgen.

Weiterhin wird vorgeschlagen, dass das Verfahren zumindest einen Reevaluationsvorgang umfasst, bei welchem vor einem Einsatz des Endoskops eine auf den hinterlegten Lage und/oder Orientierungskenngröße basierende Hand-Augen-Kalibration der Lage und/oder Orientierung des Trackermoduls relativ zur Endoskopkamera reevaluiert wird. Es kann vorteilhaft eine fehlerhafte Kalibration, eine Beschädigung von Bauteilen oder eine falsche Zuordnung von Bauteilen erkannt und vermieden werden. Ferner wird insbesondere die Hand-Augen-Kalibration unter Berücksichtigung der Kamerakenngröße reevaluiert.

Ferner wird vorgeschlagen, dass in dem Reevaluationsvorgang ein Bildsignal bereitgestellt wird, welches einem von der Endoskopkamera aufgenommenen realen Abbild eines Referenzobjekts, insbesondre des vorbenannten Referenzobjekts, entspricht. Es kann vorteilhaft eine Genauigkeit der Endoskopkamera selbst überprüft werden.

Es wird zudem vorgeschlagen, dass in dem Reevaluationsvorgang ein weiteres Bildsignal bereitgestellt wird, welches einem durch erfassen des Trackermoduls mittels einer separaten Raumkamera und anhand der hinterlegten Lage- und/oder Ortskenngrößen erzeugten virtuellen Abbild eines Referenzobjekts entspricht. Es kann vorteilhaft eine Genauigkeit der Raumkamera selbst überprüft werden. Ferner wird das virtuelle Abbild des Referenzobjekts unter Berücksichtigung der Kamerakenngröße erzeugt.

Ferner wird vorgeschlagen, dass in dem Reevaluationsvorgang zu einer Reevaluation der Hand-Augen-Kalibration ein Vergleich des Bildsignals und des weiteren Bildsignals durchgeführt wird. Es kann vorteilhaft ein bildlicher Vergleich für einen Bediener bereitgestellt werden, wodurch eine Bedienerfreundlichkeit verbessert werden kann. Insbesondere werden das Bildsignal und/oder das weitere Bildsignal auf der Anzeige des Endoskopiesystems ausgegeben. Vorzugsweise werden das Bildsignal und das weitere Bildsignal aufeinander projiziert.

Ferner wird vorgeschlagen, dass in dem Reevaluationsvorgang eine Abweichung zwischen Bildsignal und weiterem Bildsignal bestimmt wird, welche wenigstens einer Abweichung zwischen dem reellen Abbild und dem virtuellen Abbild des Referenzobjekts in zumindest einer Raumdimension entspricht. Es kann vorteilhaft eine Abweichung für einen Bediener veranschaulicht werden. Ferner wird die Abweichung insbesondere mit einer hinterlegten, insbesondere in dem Speichermedium und/oder der Speichereinheit der Steuereinheit hinterlegten, maximalen Abweichung verglichen. Überschreitet beispielsweise ein Wert der Abweichung einen Wert der maximalen Abweichung wird ein Warnsignal ausgegeben. Der Bediener wird vorzugsweise dazu aufgerufen eine andere Endoskopbasis und/oder Trackermodul zu verwenden. Ferner kann der Bediener besonders bevorzugt dazu aufgerufen werden, den Kalibriervorgang werkseitig neu durchführen zu lassen. Ganz besonders bevorzugt wird weitere Bedienung der Endoskopvorrichtung wird durch von der Steuereinheit blockiert. Insbesondere ist es denkbar, dass die Steuereinheit einen Hersteller automatisch über die vorliegende Abweichung informiert. Der Reevaluationsvorgang und insbesondere einzelne Verfahrensschritte können zur Reevaluation anhand mehrerer Abbilder, insbesondere aus verschiedenen Blickrichtungen, Blickwinkeln und/oder Entfernungen der Endoskopkamera auf das Referenzobjekt wiederholt werden.

Ferner wird vorgeschlagen, dass der Kalibiriervorgang und/oder der Reevaluationsvorgang für verschiedene Trackerebenen des Trackermoduls durchgeführt werden. Es kann vorteilhaft eine Genauigkeit weiter verbessert werden.

Die erfindungsgemäße Endoskopvorrichtung sowie das Verfahren zum Betrieb der Endoskopvorrichtung sollen hierbei nicht auf die oben beschriebene Anwendung und Ausführungsform beschränkt sein. Insbesondere kann die erfindungsgemäße Endoskopvorrichtung sowie das Verfahren zum Betrieb der Endoskopvorrichtung zu einer Erfüllung einer hierin beschriebenen Funktionsweise eine von einer hierin genannten Anzahl von einzelnen Elementen, Bauteilen und Einheiten sowie Vorgänge und Verfahrensschritte abweichende Anzahl aufweisen.

Zudem sollen bei den in dieser Offenbarung angegebenen Wertebereichen auch innerhalb der genannten Grenzen liegende Werte als offenbart und als beliebig einsetzbar gelten.

### Zeichnungen

Weitere Vorteile ergeben sich aus der folgenden Zeichnungsbeschreibung. In den Zeichnungen sind Ausführungsbeispiele der Erfindung dargestellt. Die Zeichnungen, die Beschreibung und die Ansprüche enthalten zahlreiche Merkmale in Kombination. Der Fachmann wird die Merkmale zweckmäßigerweise auch einzeln betrachten und zu sinnvollen weiteren Kombinationen zusammenfassen. Ferner sind der Übersichtlichkeit halber in den Zeichnungen und Figuren gleiche Bauteile mit gleichen Bezugszeichen versehen.

Es zeigen:
- Fig. 1: eine perspektivische Ansicht einer schematischen Darstellung eines Endoskopiesystems mit einer Endoskopvorrichtung,
- Fig. 2: eine perspektivische Ansicht einer schematischen Darstellung der Endoskopvorrichtung mit einer Endoskopbasis und einem Trackermodul, in einem verbundenen Zustand dieser,
- Fig. 3: eine distale Frontansicht einer schematischen Darstellung der Endoskopbasis,
- Fig. 4: eine perspektivische Ansicht einer schematischen Explosionsdarstellung des Trackermoduls,
- Fig. 5: eine perspektivische Ansicht einer schematischen Darstellung der Endoskopvorrichtung in einem getrennten Zustand des Trackermoduls und der Endoskopbasis,
- Fig. 6: eine proximale Rückansicht einer schematischen Darstellung des Trackermoduls
- Fig. 7: einen schematischen Ablaufplan eines beispielhaften Verfahrens zum Betrieb des Endoskopiesystems und
- Fig. 8: eine schematische Darstellung einer Draufsicht auf eine Anzeige des Endoskopiesystems, welche bei der Durchführung des Verfahrens genutzt wird und
- Fig. 9: eine alternative schematische Draufsicht einer Ausgestaltung einer Endoskopvorrichtung.

### Beschreibung der Ausführungsbeispiele

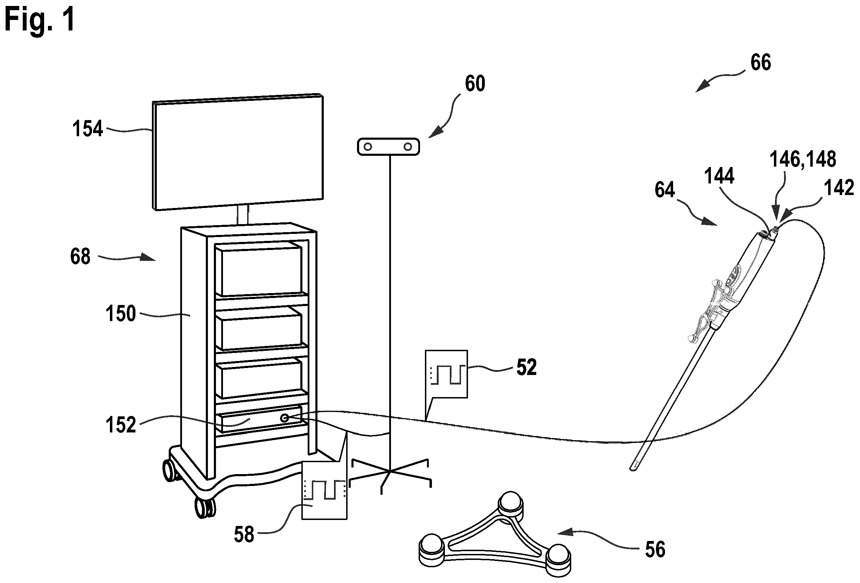

Figur 1 zeigt eine perspektivische Ansicht einer schematischen Darstellung eines Endoskopiesystems 66. Das Endoskopiesystem 66 umfasst zumindest ein Endoskop 64. Im vorliegenden Fall ist das Endoskop 64 als ein starres Videoendoskop ausgebildet und zwar insbesondere in Form eines Stereovideoendoskops. Alternativ könnte es sich bei dem Endoskop 64 auch um ein Fiberskop, Flexoskop oder dergleichen handeln.

Ferner weist das Endoskopiesystem 66 zumindest eine Endoskopvorrichtung auf. Im vorliegenden Fall umfasst die Endoskopvorrichtung das Endoskop 64.

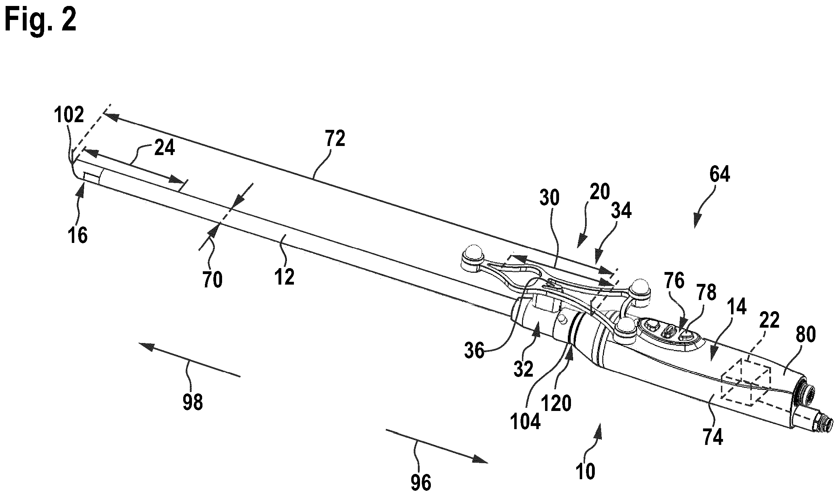

Figur 2 zeigt eine perspektivische Ansicht einer schematischen Darstellung der Endoskopvorrichtung. Die Endoskopvorrichtung umfasst zumindest eine Endoskopbasis 10. Die Endoskopbasis 10 umfasst wenigstens einen Schaft 12. Im vorliegenden Fall weist die Endoskopbasis 10 einen einzigen Schaft 12 auf. Der Schaft 12 ist zu einem Einführen in eine Körperkavität eines Patienten ausgebildet. Der Schaft 12 ist als ein längliches Bauteil ausgebildet. Der Schaft 12 weist einen runden Querschnitt auf. Alternativ könnte der Schaft 12 auch einen ovalen Querschnitt aufweisen. Der Schaft 12 weist einen Durchmesser 70 auf. Im vorliegenden Fall beträgt der Durchmesser 70 des Schafts 12 10 mm. Der Durchmesser 70 ist ein Außendurchmesser des Schafts 12. Alternativ könnte der Schaft 12 einen für einen Fachmann vorteilhaften Durchmesser 70 aufweisen, wie in etwa zumindest 2 mm und/oder höchstens 18 mm. Der Schaft 12 weist eine Haupterstreckung 72 auf. Die Haupterstreckung 72 des Schafts 12 ist zumindest um einen Faktor zehn größer als der Durchmesser 70 des Schafts 12. Der Schaft 12 ist als ein Außenschaft ausgebildet. Der Schaft 12 ist starr ausgebildet. Der Schaft 12 besteht zumindest teilweise aus Metall und zwar insbesondere aus Edelstahl.

Der Schaft 12 weist ein distales Ende 102 auf. Das distale Ende 102 ist bei einer Bedienung einem Patienten zugewandt. Das distale Ende 102 ist bei einer Bedienung einem Bediener abgewandt. Der Schaft 12 weist einen distalen Endabschnitt 24 auf. Der distale Endabschnitt 24 erstreckt sich ausgehend von dem distalen Ende 102 zu einer Mitte des Schafts 12 höchstens um 5 cm in proximaler Richtung 96. Eine Haupterstreckungsrichtung des Schafts 12 ist zumindest im Wesentlichen parallel zur proximalen Richtung 96.

Der Schaft 12 weist ein proximales Ende 104 auf. Das proximale Ende 104 ist bei einer Bedienung einem Bediener zugewandt. Das proximale Ende 104 ist bei einer Bedienung einem Patienten abgewandt. Der Schaft 12 weist einen proximalen Endabschnitt 30 auf. Der proximale Endabschnitt 30 erstreckt sich ausgehend von dem proximalen Ende 104 entlang der Haupterstreckung 72 des Schafts 12 höchstens um 5 cm in distaler Richtung 98. Die distale Richtung 98 ist der proximalen Richtung 96 entgegengesetzt. Die Haupterstreckungsrichtung des Schafts 12 ist zumindest im Wesentlichen parallel zur distalen Richtung 98.

Die Endoskopbasis 10 weist wenigstens eine Handhabe 14 auf. Im vorliegenden Fall weist die Endoskopbasis 10 eine einzige Handhabe 14 auf. Die Handhabe 14 besteht zumindest teilweise aus einem Kunststoff. Vorteilhaft ist der Kunststoff autoklavierbar. Der Schaft 12 ist fest mit der Handhabe 14 verbunden. Die Handhabe 14 ist an dem proximalen Endabschnitt 30 des Schafts 12 angeordnet. Die Handhabe 14 ist an dem proximalen Ende 104 des Schafts 12 angeordnet. Beispielsweise ist der Schaft 12 von der Handhabe 14 umspritzt. Alternativ könnte der Schaft 12 mittels eines Gewindes mit der Handhabe 14 verbunden sein.

Die Handhabe 14 weist einen Handgriff 74 auf. Im vorliegenden Fall weist die Handhabe 14 einen einzigen Handgriff 74 auf. Der Handgriff 74 ist zu einem Kontakt mit einer Hand eines Bedieners ausgebildet. Beispielsweise ist der Handgriff 74 ergonomisch an eine Kontur einer Ihn umgreifenden Hand des Bedieners angepasst. Ferner umfasst die Handhabe 14 zumindest ein Gehäuse 80. In dem Gehäuse 80 sind weitere Komponenten der Endoskopvorrichtung anordenbar. Der Handgriff 74 und das Gehäuse 80 sind zumindest teilweise einstückig verbunden. Dazu könnte der Handgriff 74 an das Gehäuse 80 angespritzt sein.

Ferner umfasst die Handhabe 14 zumindest ein Bedienmodul 76. Das Bedienmodul 76 ist zu einer Bedienung der Endoskopvorrichtung, wie beispielsweise zu einem Aktivieren und/oder Deaktivieren dieser durch den Bediener ausgebildet. Das Bedienmodul 76 umfasst zumindest ein Bedienelement 78. Das Bedienelement 78 ist als Druckknopf ausgebildet. Alternativ könnte das Bedienelement 78 auch als ein Schalter, ein berührungsempfindlicher Taster oder dergleichen ausgebildet sein.

Die Endoskopvorrichtung umfasst wenigstens eine Endoskopkamera 16. Die Endoskopkamera 16 ist zumindest teilweise in den Schaft 12 integriert. Im vorliegenden Fall ist die Endoskopkamera 16 vollständig in den Schaft 12 integriert. Die Endoskopkamera 16 ist an dem distalen Endabschnitt 24 des Schafts 12 angeordnet. Die Handhabe 14 der Endoskopbasis 10 ist frei von der Endoskopkamera 16.

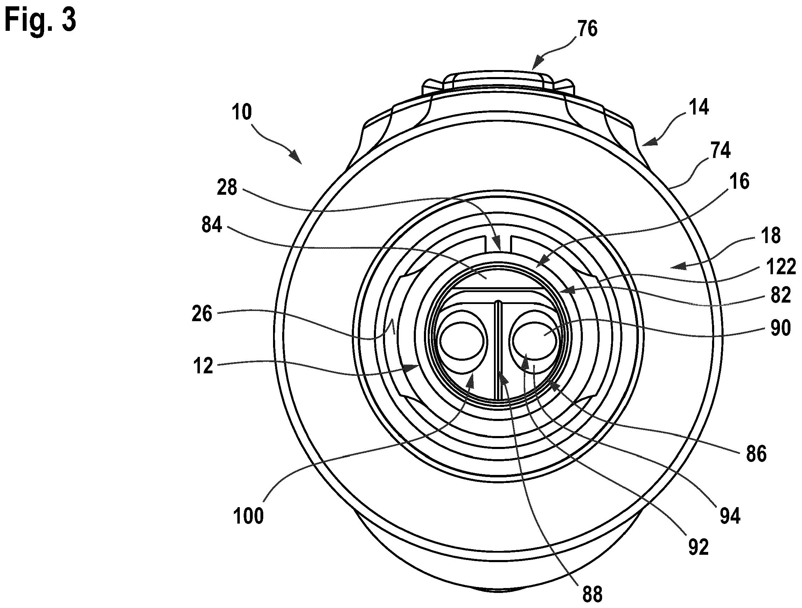

Figur 3 zeigt eine Frontansicht einer schematischen Darstellung eines Teils der Endoskopvorrichtung mit der Endoskopkamera 16. Die Endoskopkamera 16 weist ein Bilderfassungmodul 86 auf. Das Bilderfassungmodul 86 umfasst zumindest eine Bilderfassungssensorik 88. Die Bilderfassungssensorik 88 weist zumindest einen Bildsensor 90 auf. Der Bildsensor 90 ist als ein CCD-Sensor oder CMOS-Sensor ausgebildet. Ferner umfasst das Bilderfassungsmodul 86 zumindest eine Bilderfassungsoptik 92. Die Bilderfassungsoptik 92 weist zumindest ein optisches Element 94 auf, welches als ein Objektiv 94 ausgebildet ist. Ferner kann die Bilderfassungsoptik 92 weitere optische Elemente umfassen, wie beispielsweise Linsen, Prismen, Lichtwellenleiter oder dergleichen.

Die Bilderfassungsoptik 92 ist der Bilderfassungssensorik 88 zugeordnet. In proximaler Richtung 96 betrachtet ist die Bilderfassungsoptik 92 vor der Bilderfassungssensorik 88 angeordnet. Die Bilderfassungsoptik 92 ist unmittelbar an dem distalen Ende 102 des Schafts 12 angeordnet.

Im vorliegenden Fall ist die Endoskopkamera 16 als eine Stereokamera ausgebildet. Die Endoskopkamera 16 weist zumindest ein weiteres Bilderfassungmodul 100 auf. Das weitere Bilderfassungsmodul 100 ist zumindest im Wesentlichen identisch zum Bilderfassungsmodul 86 ausgebildet. Das weitere Bilderfassungsmodul 100 ist zumindest im Wesentlichen senkrecht zu einer optischen Achse des Bilderfassungsmoduls 86, insbesondere zu einer Haupterstreckungsrichtung des Schafts 12, versetzt zum Bilderfassungsmodul 86 angeordnet.

Die Endoskopvorrichtung weist zumindest ein Beleuchtungsmodul 82 auf. Das Beleuchtungsmodul 82 ist dazu ausgebildet, eine Beleuchtung für eine Bildgebung bereitzustellen. Das Beleuchtungsmodul 82 ist in dem Schaft 12 angeordnet. Das Beleuchtungsmodul 82 ist in dem distalen Endabschnitt 24 des Schafts 12 angeordnet. Das Beleuchtungsmodul 82 ist in den Schaft 12 integriert. Das Beleuchtungsmodul 82 weist zumindest ein Lichtemitter 84 auf. Bei dem Lichtemitter 84 handelt es sich um eine LED. Alternativ könnte das Beleuchtungsmodul 82 auch eine Beleuchtung über Lichtwellenleiterbündel erzielen, welche dann in dem Schaft 12 verlegt sind. Ferner kann das Beleuchtungsmodul 82 eine Beleuchtungsoptik umfassen, wie beispielsweise ein optisches Bauteil.

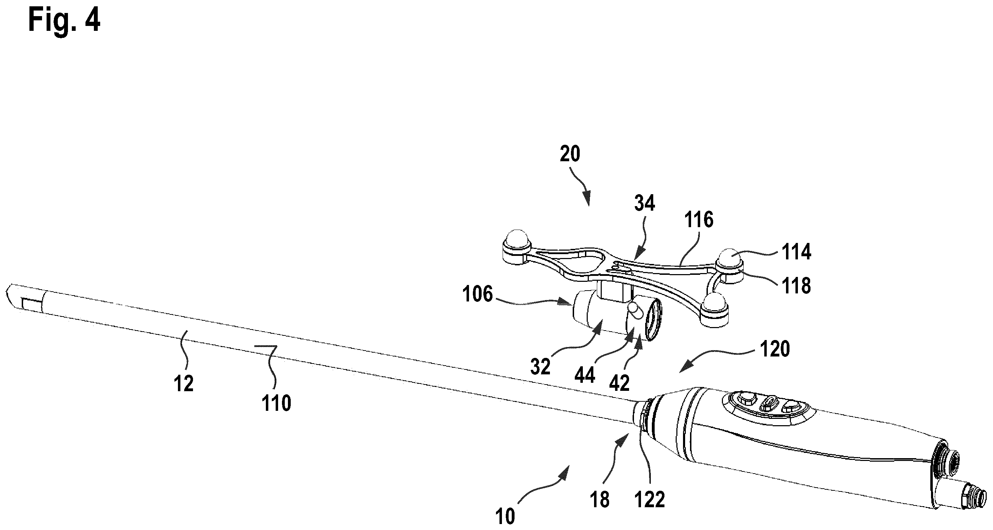

Die Endoskopvorrichtung umfasst wenigstens ein Trackermodul 20. Figuren 2 und 4 zeigen einen Teil der Endoskopvorrichtung einmal mit de Endoskopbasis 10 verbunden mit dem Trackermodul 20 und einmal mit der Endoskopbasis 10 getrennt von dem Trackermodul 20. Das Trackermodul 20 ist zu einer Bewegungsbestimmung der Endoskopvorrichtung bei einem Motion Capturing ausgebildet. Das Trackermodul 20 besteht zumindest teilweise aus Metall, insbesondere Stahl, vorzugsweise Edelstahl, und/oder Titan.

Das Trackermodul 20 ist mit dem Schaft 12 verbindbar. Das Trackermodul 20 ist auf den Schaft 12 aufschiebbar. Das Trackermodul 20 ist in proximaler Richtung 96 auf den Schaft 12 aufschiebbar. In einem verbundenen Zustand des Trackermoduls 20 umgreift dieses den Schaft 12. In einem verbunden Zustand erstreckt sich eine Haupterstreckungsrichtung des Trackermoduls 20, zumindest im Wesentlichen parallel zu einer Haupterstreckungsrichtung der Endoskopbasis 10, insbesondere des Schafts 12.

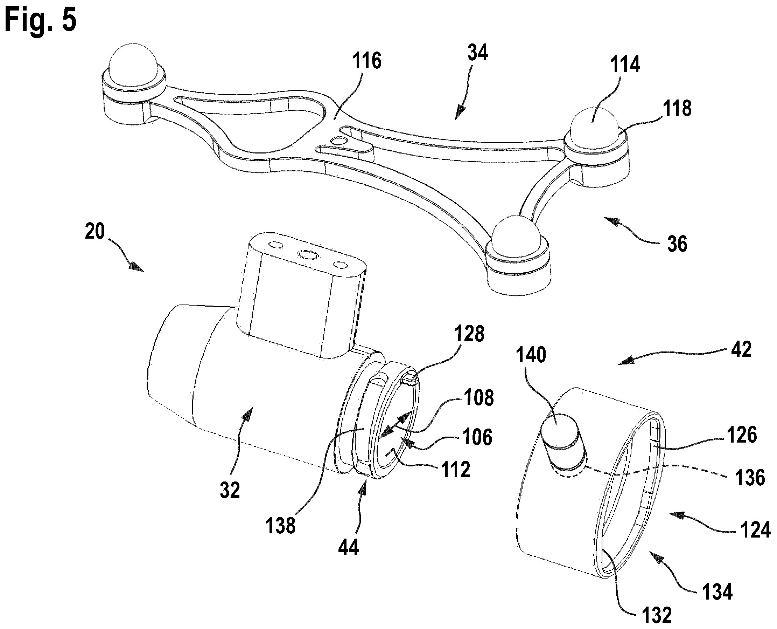

Figur 5 zeigt eine Explosionsdarstellung des Trackermoduls 20. Das Trackermodul 20 weist wenigstens eine Trackerhalterung 32 auf. Die Trackerhalterung 32 besteht zumindest teilweise aus Metall, insbesondere Stahl, vorzugsweise Edelstahl, und/oder Titan. Die Trackerhalterung 32 weist eine Ausnehmung 106, insbesondere Bohrung, auf. Bei der Ausnehmung 106 handelt es sich um eine volle Ausnehmung 106. Die Trackerhalterung 32 weist eine Form eines Hohlzylinders auf. Die Form des Hohlzylinders verengt sich konisch in distaler Richtung 98. Die Ausnehmung 106 weist einen Durchmesser 108 auf. Bei dem Durchmesser 108 handelt es sich um einen Innendurchmesser. Der Durchmesser 108 ist korrespondierende zum Durchmesser 70 des Schafts 12. Die Trackerhalterung 32 ist auf den Schaft 12 aufschiebbar. Die Trackerhalterung 32 ist auf den Schaft 12 in proximaler Richtung 96 aufschiebbar. Der Schaft 12 ist in die Ausnehmung 106 der Trackerhalterung 32 einführbar. In einem verbundenen Zustand des Trackermoduls 20 umgreift die Trackerhalterung 32 den Schaft 12 vollständig. In einem verbundenen Zustand liegen ein Mantel 110 des Schafts 12 und eine die Ausnehmung 106 begrenzende Wandung 112 der Trackerhalterung 32 aneinander an. Der Mantel 110 des Schafts 12 und die Ausnehmung 106 begrenzende Wandung 112 der Trackerhalterung 32 bilden einen Formschluss aus.

Ferner weist das Trackermodul 20 wenigstens einen Tracker 34 auf. Der Tracker 34 besteht zumindest teilweise aus Metall, insbesondere Stahl, vorzugsweise Edelstahl, und/oder Titan. Der Tracker 34 ist an der Trackerhalterung 32 angeordneten. Im vorliegenden Fall ist der Tracker 34 zumindest teilweise einstückig mit der Trackerhalterung 32 ausgebildet. Der Tracker 34 ist beispielsweise mit der Trackerhalterung 32 verschweißt. Alternativ könnte der Tracker 34 auch mit der Trackerhalterung 32 lösbar verbunden sein.

Der Tracker 34 weist im vorliegenden Fall zumindest drei Marker 114 auf. Die Marker 114 sind dazu ausgebildet, bei einem Motion Capturing erkannt zu werden. Im vorliegenden Fall sind die Marker 114 zumindest im Wesentlichen identisch ausgebildet. Die Marker 114 sind an verschiedenen Positionen des Trackers 34 angeordnet. Die Marker 114 sind als passive Marker 114 ausgebildet. Im vorliegenden Fall sind die Marker 114 als reflektierende Kugeln, insbesondere Glaskugeln, ausgebildet. Die Marker 114 sind autoklavierbar ausgebildet. Alternativ könnten die Marker 114 als aktive Marker 114 ausgebildet sein, wie beispielsweise als Leuchtdioden. Die Marker 114 sind an der Markerhalterung 116 angeordnet. Die Marker 114 sind derart angeordnet, dass diese eine Trackerebene 36 des Trackers 34 aufspannen.

Der Tracker 34 weist eine Markerhalterung 116 auf. Die Markerhalterung 116 ist zur Anordnung der Marker 114 ausgebildet. Die Markerhalterung 116 besteht zumindest teilweise aus Metall, insbesondere Stahl, vorzugsweise Edelstahl, und/oder Titan. Eine Form der Markerhalterung 116 entspricht zumindest im Wesentlichen der eines Dreiecks Die Markerhalterung 116 weist für jeden Marker 114 eine Markerstation 118 auf. Die Marker 114 sind mit den Markerstationen 118 verbunden. Die Markerstationen 118 sind in Form eines Dreiecks angeordnet. Die Markerstationen 118 sind von je einer vorzugsweise kugelschnittartigen Ausnehmung 106 der Markerhalterung 116 ausgebildet.

Die Markerhalterung 116 weist eine Haupterstreckung auf, welche sich höchstens zu einem Teil zumindest im Wesentlichen parallel entlang des Schafts 12 erstreckt. Eine Projektion der Markerhalterung 116 senkrecht zur proximal und/oder distalen Richtung 96, 98 bedeckt den Schaft 12 zu höchstens 20 % seiner Haupterstreckung 72. Ferner erstreckt sich die Haupterstreckung der Markerhalterung 116 höchstens zu einem Teil zumindest im Wesentlichen parallel zur Haupterstreckung 72 des Schafts 14. Die Markerhalterung 116 ist mit der Trackerhalterung 32 verbunden. Im vorliegenden Fall ist die Markerhalterung 116 einstückig mit der Trackerhalterung 32 verbunden. Beispielsweise kann die Markerhalterung 116 mit der Trackerhalterung 32 verschweißt sein. Alternativ könnte die Markerhalterung 116 auch mit der Trackerhalterung 32 lösbar verbunden sein, wie in etwa mittels einer Schraubverbindung.

Die Endoskopvorrichtung umfasst wenigsten einen Schnellverbinder 120 (Figur 4). Der Schnellverbinder 120 ist dazu ausgebildet, das Trackermodul 20 mit der Endoskopbasis 10 form- und/oder kraftschlüssig zu verbinden. Der Schnellverbinder 120 weist zumindest eine Schnellverbinderschnittstelle 18 auf. Die Schnellverbinderschnittstelle 18 besteht insbesondere zumindest teilweise, vorzugsweise zumindest zu einem Großteil und besonders bevorzugt vollständig aus Metall, insbesondere Stahl, vorzugsweise Edelstahl, und/oder Titan. Die Schnellverbinderschnittstelle 18 definiert wenigstens eine Lage und/oder Orientierung des Trackermoduls 20 relativ zur Endoskopkamera 16. Die Schnellverbinderschnittstelle 18 ist zu einer lösbaren Verbindung wenigstens des Trackermoduls 20 mit der Endoskopbasis 10 ausgebildet. Die Schnellverbinderschnittstelle 18 ist zumindest teilweise an einem proximalen Endabschnitt 30 des Schafts 12 angeordnet. Die Schnellverbinderschnittstelle 18 ist fest mit dem Schaft 12 verbunden. Die Schnellverbinderschnittstelle 18 kann beispielsweise mit dem Schaft 12 verschweißt sein.

Die Schnellverbinderschnittstelle 18 weist zumindest ein Kragenelement 122 auf. Das Kragenelement 122 erstreckt sich zumindest im Wesentlichen senkrecht zu einer Haupterstreckungsrichtung der Endoskopbasis 10. Das Kragenelement 122 umgreift insbesondere den Schaft 12 der Endoskopbasis 10. Das Kragenelement 122 ist einstückig mit dem Schaft 12 verbunden.

Die Schnellverbinderschnittstelle 18 weist wenigstens einen stirnseitigen Anschlag 26 auf (siehe Figur 3). Der Anschlag 26 ist dazu ausgebildet, die Lage des Trackermoduls 20 relativ zur Endoskopkamera 16 festzulegen. Der stirnseitige Anschlag 26 ist von dem Kragenelement 122 ausgebildet. Der Anschlag 26 ist von einer Stirn des Kragenelements 122 gebildet.

Die Schnellverbinderschnittstelle 18 weist zumindest ein Poka-Yoke-Element 28 auf. Das Poka-Yoke-Element 28 ist dazu ausgebildet, die Orientierung des Trackermoduls 20 relativ zur Endoskopkamera 16 festzulegen. Das Poka-Yoke-Element 28 ist im vorliegenden Fall als eine Nut ausgebildet. Das Poka-Yoke-Element 28 ist von dem Kragenelement 122 ausgebildet. Das Poka-Yoke-Element 28 ist von einer im Mantel 110 des Kragenelements 122 angeordneten Nut ausgebildet. Alternativ könnte das Poka-Yoke-Element 28 auch als ein Pin ausgebildet sein.

Der Schnellverbinder 120 weist zumindest eine weitere Schnellverbinderschnittstelle 42 auf. Die weitere Schnellverbinderschnittstelle 42 ist von dem Trackermodul 20 bereitgestellt. Die weitere Schnellverbinderschnittstelle 42 ist korrespondierend zur Schnellverbinderschnittstelle 18 ausgebildet. Bei einer Verbindung des Trackermoduls 20 mit der Endoskopbasis 10 wirken die Schnellverbinderschnittstelle 18 und die weitere Schnellverbinderschnittstelle 42 zusammen.

Die weitere Schnellverbinderschnittstelle 42 weist zumindest ein weiteres Kragenelement 124 auf. Das weitere Kragenelement 124 ist korrespondierend zum Kragenelement 122 ausgebildet. Das weitere Kragenelement 124 ist drehbar gelagert. Das weitere Kragenelement 124 erstreckt sich zumindest im Wesentlichen senkrecht zu einer Haupterstreckungsrichtung der Endoskopbasis 10. Das weitere Kragenelement 124 umgreift den Schaft 12. Das weitere Kragenelement 124 ist einstückig mit dem Trackermodul 20 verbunden.

Die weitere Schnellverbinderschnittstelle 42 weist wenigstens einen weiteren Anschlag 126 auf. Der weitere Anschlag 126 ist stirnseitig. Der weitere Anschlag 126 ist dazu ausgebildet, die Lage des Trackermoduls 20 relativ zur Endoskopkamera 16 zusammen mit dem Anschlag 26 festzulegen. Der stirnseitige Anschlag 26 ist von dem weiteren Kragenelement 124 ausgebildet. Der weitere Anschlag 126 ist von einer Stirn des weiteren Kragenelements 124 gebildet. In einem verbunden Zustand liegen die Anschläge 26, 126 stirnseitig aneinander an.

Die Schnellverbinderschnittstelle 18 weist zumindest ein weiteres Poka-Yoke-Element 128 auf. Das weitere Poka-Yoke-Element 128 ist dazu ausgebildet, die Orientierung des Trackermoduls 20 relativ zur Endoskopkamera 16 festzulegen. Das weitere Poka-Yoke-Element 128 ist korrespondierend zum Poka-Yoke-Element 28 ausgebildet. Das weitere Poka-Yoke-Element 128 ist im vorliegenden Fall als ein Pin ausgebildet. Das weitere Poka-Yoke-Element 128 ist von dem Trackermodul 20 bereitgestellt. Das weitere Poka-Yoke-Element 128 ist von dem Mantel 110 der Trackerhalterung 32 ausgebildet.

Die Schnellverbinderschnittstelle 18 weist eine Verbindungsform 130 auf. Die Verbindungsform 130 ist stirnseitig. Die Verbindungsform 130 ist von dem Kragenelement 122 ausgebildet. Die Verbindungsform 130 weist eine zumindest zweifache Drehsymmetrie aufweist. Die Verbindungsform 130 weist jedoch keine Rotationssymmetrie auf. Die Verbindungsform 130 weist in einer Draufsicht, insbesondere in proximaler Richtung 96, eine Formgebung eines Vierecks mit an zwei gegenüberliegenden gerundeten Seiten auf.

Ferner weist die weitere Schnellverbinderschnittstelle 42 eine weitere Verbindungsform 132 auf. Die weitere Verbindungsform 132 ist korrespondierend zur Verbindungsform 130 ausgebildet. Die Verbindungsformen 130, 132 sind in der Art eines Schlüssels und eines Schlüssellochs zueinander korrespondierend ausgebildet. Die Verbindungsform 130 ist von dem weiteren Kragenelement 124 ausgebildet.

Die Schnellverbinderschnittstellen 18, 42 weisen zueinander eine erste Anordnung auf. In der ersten Anordnung sind die Schnellverbinderschnittstellen 18, 42 zueinander gleich ausgerichtet. In der ersten Anordnung beträgt ein Winkel zwischen den Schnellverbinderschnittstellen 18, 42 0°. Alternativ könnte ein Winkel zwischen den Schnellverbinderschnittstellen 18, 42 180° betragen. In der ersten Anordnung sind die Schnellverbinderschnittstellen 18, 42 derart zueinander ausgerichtet, dass die Verbindungsformen 130, 132 der Schnellverbinderschnittstellen 18, 42 und zwar insbesondere deren Verbindungsformen 130, 132 zueinander kongruent angeordnet. Die Schnellverbinderschnittstellen 18, 42 sind in der ersten Anordnung stirnseitig ineinander einführbar.

Die Schnellverbinderschnittstellen 18, 42 weisen zueinander eine zweite Anordnung auf. In der zweiten Anordnung sind die Schnellverbinderschnittstellen 18, 42 und zwar insbesondere deren Verbindungsformen 130, 132 zueinander verschränkt ausgerichtet. In der zweiten Anordnung beträgt ein Winkel zwischen den Schnellverbinderschnittstellen 18, 42 90°. Alternativ könnte ein Winkel zwischen den Schnellverbinderschnittstellen 18, 42 270° betragen. Um die Schnellverbinderschnittstellen 18, 42 von der ersten Anordnung in die zweite Anordnung zu überführen sind diese um 90° zueinander verdrehbar. In der zweiten Anordnung der Schnellverbinderschnittstellen 18, 42 zueinander, liegen die Schnellverbinderschnittstellen 18, 42 stirnseitig aneinander an.

Die Endoskopvorrichtung weist zumindest einen Schnellspanner 44 auf. Der Schnellspanner 44 verspannt die Schnellverbinderschnittstellen 18, 42 bei einer Verbindung des Trackermoduls 20 mit der Endoskopbasis 10 durch Kraftbeaufschlagung miteinander. Der Schnellspanner 44 ist zumindest teilweise einstückig mit dem Trackermodul 20 ausgebildet. Ferner ist der Schnellspanner 44 zumindest teilweise einstückig mit dem Schnellverbinder 120, insbesondere der weiteren Schnellverbinderschnittstelle 42 des Schnellverbinders 120 ausgebildet. Der Schnellverbinder 120 besteht zumindest teilweise aus Metall, insbesondere Stahl, vorzugsweise Edelstahl, und/oder Titan.

Der Schnellspanner 44 weist eine erste Position auf. Ferner weist der Schnellspanner 44 eine zweite Position auf. Der Schnellspanner 44 ist von der ersten Position in die zweite Position überführbar und umgekehrt. In der ersten Position des Schnellspanners 44 sind die Schnellverbinderschnittstellen 18, 42 voneinander gelöst. In der zweiten Position des Schnellspanners 44 sind die Schnellverbinderschnittstellen 18, 42 stirnseitig ineinander einführbar. Der Schnellspanner 44 ist durch verdrehen von der ersten Position in die zweite Position überführbar und umgekehrt und zwar insbesondere um einen Winkel von 90°. In der zweiten Position verspannt der Schnellspanner 44 die Schnellverbinderschnittstellen 18, 42 miteinander. In der zweiten Position des Schnellspanners 44 liegen die Anschläge 26, 126 der Schnellverbinderschnittstellen 18, 42 fest aneinander an. In der zweiten Position des Schnellspanners 44 sind die Anschläge 26, 126 mit Kraft beaufschlagt. Die erste Position des Schnellspanner 44 entspricht der ersten Anordnung der Schnellverbinderschnittstellen 18, 42. Die zweite Position des Schnellspanners 44 entspricht der zweiten Anordnung der Schnellverbinderschnittstellen 18, 42. Die Anordnungen der Schnellverbinder 120 sind unmittelbar mit den Positionen des Schnellspanners 44 verknüpft.

Der Schnellspanner 44 weist zumindest einen Spannring 134 auf. Der Spannring 134 ist um den Schaft 12 drehbar gelagert. Der Spannring 134 ist einstückig mit dem weiteren Kragenelement 124 der weiteren Schnellverbinderschnittstelle 42 einstückig verbunden. Mittels des Spannrings 134 ist der Schnellspanner 44 von der ersten Position in die zweite Position überführbar und umgekehrt.

Der Schnellspanner 44 weist zumindest ein Klemmelement 136 auf. Das Klemmelement 136 definiert zumindest die zweite Position des Schnellspanners 44. Mittels des Klemmelements 136 ist der Schnellspanner 44 in der zweiten Position verklemmbar. Im vorliegenden Fall ist das Klemmelement 136 als ein Gewinde ausgebildet. Das Klemmelement 136 dient zu einer Definierung der Position des Schnellspanners 44. Das Klemmelement 136 ist von einer Ausnehmung 106 der Trackerhalterung 32 ausgebildet.

Zudem weist der Schnellspanner 44 ein weiteres Klemm- und/oder Spannelement 138 auf. Das weitere Klemm- und/oder Spannelement 138 ist korrespondierend zu dem Klemm- und/oder Spannelement 136 ausgebildet. Das weitere Klemm- und/oder Spannelement 138 ist von dem Klemm- und/oder Spannelement 136 geführt. Im vorliegenden Fall ist das weitere Klemm- und/oder Spannelement 138 als ein Zapfen ausgebildet. Das weitere Klemm- und/oder Spannelement 138 ist an dem Spannring 134 angeordnet. Durch Führung des weiteren Klemm- und/oder Spannelement 138 in dem Klemm- und/oder Spannelement 136 kann die erste Position in die zweite Position des Schnellspanner 44 und insbesondere die erste Anordnung der Schnellverbinderschnittstellen 18 in die zweite Anordnung, überführt werden.

Zu einer Betätigung des Schnellspanners 44 weist dieser ein Betätigungselement 140 auf. Das Betätigungselement 140 ist an dem Spannring 134 angeordnet. Das Betätigungselement 140 ist stiftförmig ausgebildet. Das Betätigungselement 140 ist einstückig mit dem weiteren Klemm- und/oder Spannelement 138 ausgebildet.

Die Endoskopvorrichtung weist zumindest ein elektrisches und/oder elektronisches Schnittstellenmodul 142 auf (siehe Figur 1). Das elektrische und/oder elektronische Schnittstellenmodul 142 ist zu einer elektrischen und/oder elektronischen Verbindung der Endoskopvorrichtung mit weiteren Komponenten des Endoskopiesystems 66 ausgebildet. Das elektrische und/oder elektronische Schnittstellenmodul 142 ist zumindest teilweise in der Endoskopbasis 10 angeordnet. Das elektrische und/oder elektronische Schnittstellenmodul 142 ist in das von der Handhabe 14 bereitgestellte Gehäuse 80 integriert. Das elektrische und/oder elektronische Schnittstellenmodul142 weist zumindest einen Anschluss 144 auf. Im vorliegenden Fall ist der Anschluss 144 kabelgebunden. Der Anschluss 144 kann dabei als USB-, BUS-, CAN- und/oder LAN-Anschluss ausgebildet sein.

Das elektrische und/oder elektronische Schnittstellenmodul 142 umfasst zumindest eine Energieschnittstelle 146. Die Energieschnittstelle 146 ist zur Versorgung von weiteren Komponenten der Endoskopvorrichtung mit elektrischer Energie ausgebildet. Die Energieschnittstelle 146 ist von dem Anschluss 144 ausgebildet.

Das elektrische und/oder elektronische Schnittstellenmodul 142 umfasst zumindest eine Datenschnittstelle 148. Die Datenschnittstelle 148 ist zum Austausch von Daten, insbesondere einer Lage- und/oder der Orientierungskenngröße und/oder Kamerakenngröße, mit weiteren Komponenten der Endoskopvorrichtung und/oder des Endoskopiesystems 66 ausgebildet. Die Datenschnittstelle 148 ist von dem Anschluss 144 ausgebildet. Die Datenschnittstelle 148 und die Energieschnittstelle 146 sind im vorliegenden Fall einstückig ausgebildet.

Das Endoskopiesystem 66 umfasst wenigstens ein der Endoskopvorrichtung zugeordnetes Speichermedium 22 (siehe Figur 2). Auf dem zugeordneten Speichermedium 22 ist wenigstens eine werkseitig bestimmte Lage- und/oder Orientierungskenngröße der definierten Lage und/oder Orientierung des Trackermoduls 20 relativ zur Endoskopkamera 16 wiederabrufbar hinterlegt. Ferner ist zumindest eine Kamerakenngröße der Endoskopkamera 16 wiederabrufbar auf dem Speichermedium 22 hinterlegt. Im vorliegenden Fall ist als eine Lagekenngröße zumindest ein Abstand der Endoskopkamera 16 relativ zum Trackermodul 20 hinterlegt. Ferner ist als eine Orientierungskenngröße zumindest ein Polarwinkel der Endoskopkamera 16 relativ zum Trackermodul 20 hinterlegt. Ferner ist als eine weitere Orientierungskenngröße zumindest ein Azimutwinkel der Endoskopkamera 16 relativ zum Trackermodul 20 hinterlegt. Zudem ist als zumindest eine Kamerakenngröße zumindest eine Brennweite der Endoskopkamera 16 hinterlegt. Ferner könnten weitere Kameraparameter hinterlegt sein, wie in etwa deren Verzeichnung, Bildzentrum, Schärfentiefe oder dergleichen. Bei dem Speichermedium 22 handelt es sich im vorliegenden Fall um einen Massendatenspeicher. Ferner sind Ausgestaltungen des Speichermediums 22 als ein von einem Server, insbesondere einem Cloud-Server, bereitgestellter Speicherabschnitt denkbar.

Im vorliegenden Fall ist das Speichermedium 22 Teil der Endoskopvorrichtung. Das Speichermedium 22 ist in die Endoskopbasis 10, insbesondere die Handhabe 14, integriert. Das Speichermedium 22 ist in das von der Handhabe 14 bereitgestellte Gehäuse 80 integriert. Alternativ könnte das Speichermedium 22 auch separat von der Endoskopvorrichtung ausgebildet sein.

Auf dem Speichermedium 22 ist wenigstens eine Kennung, wie etwa einem Schlüssel, insbesondere einer Seriennummer, des Speichermediums 22 und/oder der Endoskopvorrichtung der Endoskopvorrichtung hinterlegt. Die Kennung ist dazu ausgebildet, das Speichermedium 22 der Endoskopvorrichtung zuzuordnen. Ferner ist das Speichermedium 22 basierend auf der Kennung, verschlüsselt ausgebildet, so dass ein Zugriff auf dieses ausschließlich bei einer passenden Zuordnung des Speichermediums 22 zu einer vorgesehenen Endoskopvorrichtung möglich ist.

Das Endoskopiesystem 66 weist zumindest eine Raumkamera 60 auf (siehe Figur 1). Die Raumkamera 60 ist separat von der Endoskopvorrichtung ausgebildet. Die Raumkamera 60 ist relativ zu der Endoskopvorrichtung unbeweglich. Die Raumkamera 60 bildet einen festen Bezugspunkt aus. Die Raumkamera 60 erfasst das Trackermodul 20. Die Raumkamera 60 ist dazu ausgebildet, die Lage- und/oder Orientierung des Trackermoduls 20 relativ zum Endoskopkamera 16 zu erfassen.

Ferner weist das Endoskopiesystem 66 zumindest ein Referenzobjekt 56 auf. Bei dem Referenzobjekt 56 handelte es sich im vorliegenden Fall um einen weiteren Tracker. Alternativ könnte es sich bei dem Referenzobjekt 56 um ein Feld handeln, welches ein Muster umfasst, wie beispielsweise ein Schachbrettmuster oder dergleichen.

Ferner weist das Endoskopiesystem 66 zumindest eine Steuereinheit 68 auf. Die Steuereinheit 68 ist zur Durchführung eines Verfahrens zum Betrieb der Endoskopvorrichtung ausgebildet. Die Steuereinheit 68 ist in einem Rack 150 des Endoskopiesystems 66 angeordnet. Alternativ könnte die Endoskopvorrichtung selbst eine Steuereinheit 68 umfassen, welche insbesondere in die Endoskopbasis 10, vorzugsweise in die Handhabe 14 und besonders bevorzugt in ein von der Handhabe 14 bereitgestelltes Gehäuse 80 integriert sein könnte.

Im vorliegenden Fall ist die Steuereinheit 68 als ein Computer ausgebildet. Alternativ oder zusätzlich könnte es sich bei der Steuereinheit 68 um ein Tablet, ein Smartphone oder dergleichen handeln. Die Steuereinheit 68 umfasst zumindest eine Steuerelektronik 152. Komponenten der Steuerelektronik 152 sind der Übersichtlichkeit halber in den Figuren nicht dargestellt. Die Steuerelektronik 152 umfasst einen Prozessor. Ferner umfasst die Steuerelektronik 152 einer Speichereinheit, wie beispielsweise eine Festplatte. Auf der Speichereinheit ist zumindest ein Betriebsprogramm hinterlegt. Das Betriebsprogramm ist von dem Prozessor ausführbar.

Ferner umfasst das Endoskopiesystem 66 zumindest eine Anzeige 154, wie beispielsweise einen Computerbildschirm. Die Anzeige 154 ist in dem Rack 150 angeordnet. Die Anzeige 154 ist dazu ausgebildet, ein von der Endoskopkamera 16 bereitgestelltes Bildsignal 52 anzuzeigen. Ferner ist die Anzeige 154 dazu ausgebildet, Informationen des Betriebsprogramms darzulegen.

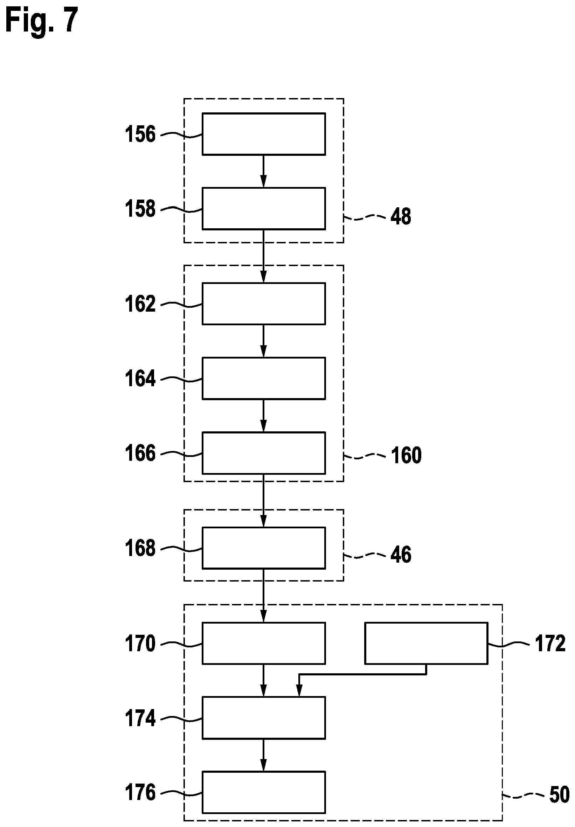

Figur 7 zeigt einen schematischen Ablaufplan eines Verfahrens zum Betrieb des Endoskopiesystems 66. Das Verfahren ist Teil des Betriebsprogamms.

Das Verfahren umfasst zumindest einen Testvorgang 48. In dem Testvorgang 48 wird ein Abgleich einer vorliegenden Komponentenzuordnung durchgeführt. Der Testvorgang 48 umfasst zumindest einen Verfahrensschritt 156. In dem Verfahrensschritt 156 wird eine vorliegende Endoskopbasis 10 identifiziert. Im vorliegenden Fall wird die Endoskopbasis 10 anhand einer Kennung, wie beispielsweise einer Seriennummer identifiziert. Alternativ könnte eine Identifizierung elektrisch und/oder elektronisch erfolgen, wie beispielsweise über die elektrische und/oder elektronische Schnittstelle oder aber auch über einen an der Endoskopbasis 10 angeordneten RFID-Chip. Ferner wir eine vorliegendes Trackermodul 20 identifiziert. Dieses kann korrespondierend zum Endoskopbasis 10 identifiziert werden.

Der Testvorgang 48 umfasst zumindest einen weiteren Verfahrensschritt 158. In dem weiteren Verfahrensschritt 158 wird die vorliegenden identifizierten Komponenten und zwar das Trackermodul 20 und die Endoskopbasis 10 mittels der Steuereinheit 68 angemeldet. In der Steuereinheit 68 wird die Kennungen der Endoskopbasis 10 und des Trackermoduls 20 abgeglichen um eine vorgesehen Komponentenzuordnung zu ermitteln. Für den Fall, dass das vorliegende Trackermodul 20 und die Endoskopbasis 10 von einer vorgesehen Komponentenzuordnung abweichen, wird das Verfahren abgebrochen. Alternativ könnte eine Fehlermeldung ausgegeben werden, beispielsweise mittels der Anzeige 154, und eine neue Zuordnung angefordert werden, wonach der vorliegende Testvorgang 48 wiederholt wird. Für den Fall, dass das vorliegende Trackermodul 20 und die Endoskopbasis 10 einer vorgesehen Komponentenzuordnung entsprechen, wird das Verfahren fortgesetzt. Denkbar ist, dass auf den Testvorgang 48 bei Durchführung des vorliegenden Verfahrens verzichtet werden kann.

Das Verfahren umfasst wenigstens einen Montagevorgang 160. In dem Montagevorgang 160 werden die vorgesehenen Komponente, die Endoskopbasis 10 und das Trackermodul 20 miteinander verbunden. Der Montagevorgang 160 umfasst zumindest einen Verfahrensschritt 162. In dem Verfahrensschritt 162 wird das Trackermodul 20 auf die Endoskopbasis 10 geschoben. Dazu wird der Schaft 12 in die Ausnehmung 106 der Trackerhalterung 32 eingeführt. Das Trackermodul 20 wird relativ zur Endoskopbasis 10 in proximaler Richtung 96 geschoben. Das Trackermodul 20 wird bis zum proximalen Endabschnitt 30 des Schafts 12 geschoben. Am proximalen Endabschnitt 30 des Schafts 12 liegen die Schnellverbinderschnittstellen 18, 42 des Schnellverbinders 120 aneinander an.

Der Montagevorgang 160 umfasst zumindest einen weiteren Verfahrensschritt 164. In dem weiteren Verfahrensschritt 164 werden die Schnellverbinderschnittstellen 18, 42 zueinander in die erste Anordnung gebracht. Ferner werden die Poka-Yoke-Elemente 28, 128 ineinander eingeführt. Diese legen dann eine Orientierung des Trackermoduls 20 relativ zur Endoskopkamera 16 fest. Die weitere Schnellverbinderschnittstelle 42 wird dazu verdreht bis die erste Anordnung hergestellt ist. In der ersten Anordnung greifen die Schnellverbinderschnittstellen 18, 42 stirnseitig ineinander ein. Da der Spannring 134 einstückig mit der weiteren Schnellverbinderschnittstelle 42 ausgebildet wird, wird dieser dabei automatisch auch in die erste Position gebracht. Das Trackermodul 20 wird in proximaler Richtung 96 relativ zur Endoskopbasis 10 weiter verschoben bis sich die Schnellverbinderschnittstellen 18, 42 hintergreifen.

Der Montagevorgang 160 umfasst zumindest einen weiteren Verfahrensschritt 166. In dem weiteren Verfahrensschritt 166 werden die Schnellverbinderschnittstellen 18, 42 von der ersten Anordnung in die zweite Anordnung überführt. Ferner wird der Schnellspanner 44 von der ersten Position in die zweite Position überführt. Dazu werden die Schnellverbinderschnittstellen 18, 42 zueinander verdreht. Die Verbindungsformen 130, 132 der Schnellverbinderschnittstellen 18, 42 werden zueinander verschränkt. Die Schnellverbinderschnittstellen 18, 42 liegen stirnseitig aneinander an. Durch gleichzeitige verdrehen des Schnellspanners 44 von der ersten Position in die zweite Position werden die Schnellverbinderschnittstellen 18, 42 miteinander verspannt. Die Schnellverbinderschnittstellen 18, 42 liegen aneinander an, so dass deren Anschläge eine Lage der Trackermoduls 20 relativ zur Endoskopkamera 16 festlegen. Demnach ist bei einer hergestellten Verbindung mittels des Schnellverbinders 120 die Lage und/oder Orientierung des Trackermoduls 20 relativ zur Endoskopbasis 10 definiert.

Das Verfahren umfasst wenigstens einen Kalibriervorgang 46. Der Kalibriervorgang 46 wird werkseitig ausgeführt. In dem Kalibriervorgang 46 wird werkseitig wenigstens eine Lage- und/oder Orientierungskenngröße der von der Schnellverbinderschnittstelle 18 definierten Lage und/oder Orientierung des Trackermoduls 20 relativ zur Endoskopkamera 16 bestimmt.

Der Kalibriervorgang 46 umfasst zumindest einen Verfahrensschritt 168. In dem Verfahrensschritt 168 wird eine Hand-Augen-Kalibration durchgeführt, mittels welcher die Lage- und/oder Orientierungskenngröße bestimmt werden. Die Lage- und/oder Orientierungskenngröße wird wiederabrufbar auf dem Speichermedium 22 hinterlegt. Ferner könnten mittels der Hand-Auge-Kalibration auch die Kamerakenngröße bestimmt und vorteilhaft wiederabrufbar in dem Speichermedium 22 hinterlegt werden.

Das Verfahren umfasst zumindest einen Reevaluationsvorgang 50. Der Reevaluationsvorgang 50 wird vor einem Einsatz der Endoskopvorrichtung von einem Bediener durchgeführt. In dem Reevaluationsvorgang 50 wird die auf den hinterlegten Lage und/oder Orientierungskenngröße basierende Hand-Augen-Kalibration der Lage und/oder Orientierung des Trackermoduls 20 relativ zur Endoskopkamera 16 reevaluiert.

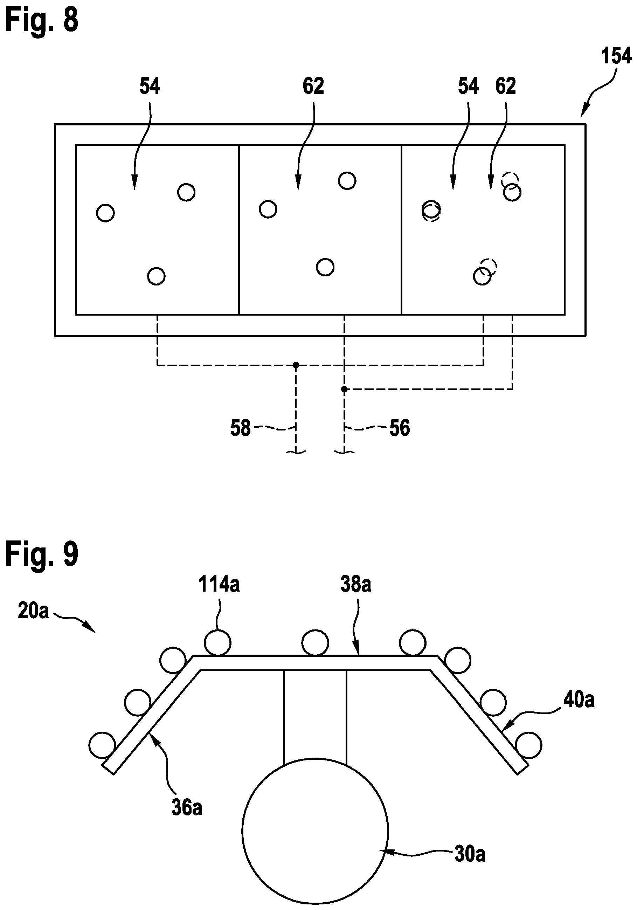

Der Reevaluationsvorgang 50 umfasst zumindest einen Verfahrensschritt 170. In dem Verfahrensschritt 170 wird von der Endoskopkamera 16 das Referenzobjekt 56 aufgenommen. Die Endoskopkamera 16 stellt ein Bildsignal 52 bereit, welches einem von der Endoskopkamera 16 aufgenommenen realen Abbild 54 des Referenzobjekts 56 entspricht (siehe Figur 8).

Der Reevaluationsvorgang 50 umfasst zumindest einen weiteren Verfahrensschritt 172. Der weitere Verfahrensschritt 172 erfolgt im Wesentlichen zeitgleich mit dem Verfahrensschritt 170. In weiteren Verfahrensschritt 172 wird ein weiteres Bildsignal 58 bereitgestellt. Das weitere Bildsignal 58 wird von der Steuereinheit 68 bereitgestellt. Mittels der separaten Raumkamera 60 wird die Lage- und/oder Orientierung des Trackermoduls 20 bestimmt. Die Raumkamera 60 erfasst das Trackermodul 20. Die Steuereinheit 68 ruft die Lage- und/oder Orientierungskenngröße und/oder die Kamerakenngröße des Trackermoduls 20 zum Berechnen des weiteren Bildsignals 58 ab. Das weitere Bildsignal 58 entspricht einem virtuellen Abbild 62 des Referenzobjekts 56.

Der Reevaluationsvorgang 50 umfasst zumindest einen weiteren Verfahrensschritt 174. In dem weiteren Verfahrensschritt 174 wird zu einer Reevaluation der Hand-Augen-Kalibration des Kalibriervorgangs 46 ein Vergleich des Bildsignals 52 und des weiteren Bildsignals 58 durchgeführt. Das Bildsignal 52 wird auf der Anzeige 154 dargestellt. Ferne wird das weitere Bildsignal 58 auf der Anzeige 154 dargestellt. Die Steuereinheit 68 erzeugt zu einem visuellen Vergleich einer Projektion des Bildsignals 52 und des weiteren Bildsignal 58 aufeinander. Ferner wird eine Abweichung zwischen Bildsignal 52 und weiterem Bildsignal 58 bestimmt. Die Abweichung entspricht wenigstens einer Abweichung zwischen dem reellen Abbild 54 und dem virtuellen Abbild 62 des Referenzobjekts 56 in zumindest zwei Raumdimensionen. Insbesondere bei einem Trackermodul 20, welches mehrere Trackerebenen 36, 38, 40 umfasst, ist eine bestimmte Abweichung in zumindest drei Raumdimensionen denkbar.

Der Reevaluationsvorgang 50 umfasst zumindest einen weiteren Verfahrensschritt 176. In dem weiteren Verfahrensschritt 176 wird die Abweichung mit einer hinterlegten maximalen Abweichung verglichen. Überschreitet ein Wert der Abweichung einen Wert der maximalen Abweichung wird ein Warnsignal ausgegeben. Der Bediener wird dazu aufgerufen eine andere Endoskopbasis 10 und/oder Trackermodul 20 zu verwenden. Ferner kann der Bediener dazu aufgerufen werden den Kalibriervorgang 46 werkseitig neu durchführen zu lassen. Ferner ist denkbar, dass eine weitere Bedienung der Endoskopvorrichtung von der Steuereinheit 68 blockiert wird.

Der Reevaluationsvorgang 50, insbesondere einzelne Verfahrensschritte des Reevaluationsvorgangs 50, können zur Reevaluation anhand mehrere Abbilder, insbesondere aus verschiedenen Blickrichtungen, Blickwinkeln und/oder Entfernungen der Endoskopkamera 16 auf das Referenzobjekt 56 wiederholt werden.

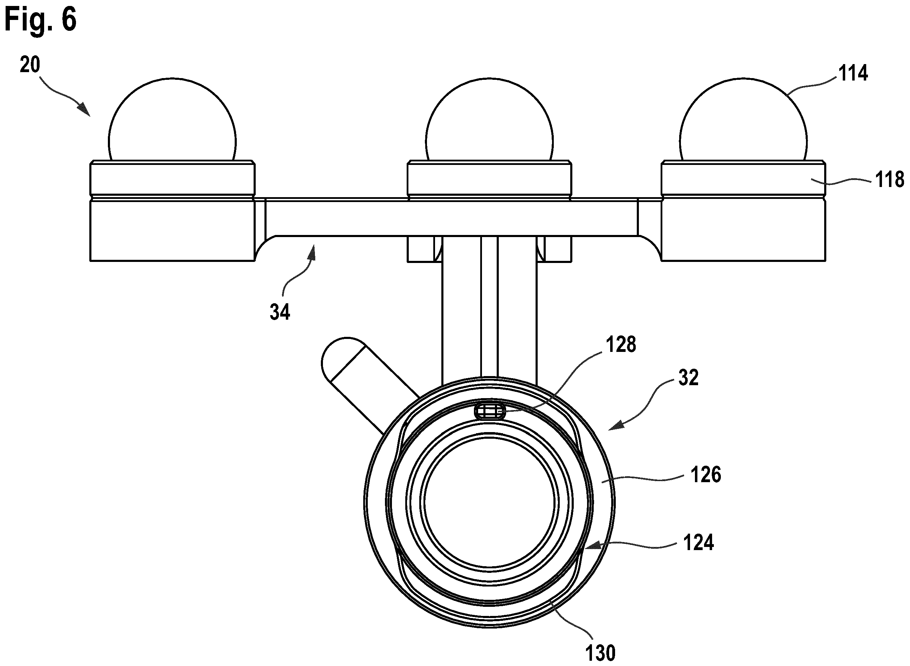

In den Figur 9 ist ein weiteres Ausführungsbeispiel der Erfindung gezeigt. Die nachfolgenden Beschreibungen und die Zeichnungen beschränken sich im Wesentlichen auf die Unterschiede zwischen den Ausführungsbeispielen, wobei bezüglich gleich bezeichneter Bauteile, insbesondere in Bezug auf Bauteile mit gleichen Bezugszeichen, grundsätzlich auch auf die Zeichnungen und/oder die Beschreibung des anderen Ausführungsbeispiels, insbesondere der Figuren 1 bis 8, verwiesen werden kann. Zur Unterscheidung der Ausführungsbeispiele ist in dem Ausführungsbeispiel der Figur 9 ein jeweiliges Bezugszeichen durch den Buchstaben a ergänzt.

Figur 9 zeigt eine alternative Ausgestaltung des Endoskopiesystems 66 mit einer Endoskopvorrichtung. Das vorliegende Ausführungsbeispiel unterscheidet sich im Wesentlichen von dem vorhergehenden durch eine Ausgestaltung eines Trackermoduls 20a der Endoskopvorrichtung. Das Trackermodul 20a weist wenigstens zwei verschiedene Trackerebenen 36a, 38a, 40a auf. Das Trackermodul 20a weist eine erste Trackerebene 36a auf. Ferner weist das Trackermodul 20a eine zweite Trackerebene 38a auf. Zudem weist das Trackermodul 20a eine dritte Trackerebene 40a auf. Im vorliegenden Fall weist das Trackermodul 20a somit sogar drei verschiedene Trackerebenen 36a, 38a, 40a auf. Die Trackerebenen 36a, 38a, 40a sind zueinander winklig angeordnet. Die Trackerebenen 36a, 38a, 40a sind von verschiedenen Markern 114a des Trackermoduls 20a aufgespannt. Keine der Trackerebenen 36a, 38a, 40a teilt sich einen Marker 114a mit einer weiteren Trackerebene 36a, 38a, 40a. Die Trackerebenen 36a, 38a, 40a sind zueinander in Umfangsrichtung eines Schafts 12 der Endoskopvorrichtung versetzt zueinander angeordnet.

Das Trackermodul 20a weistje Trackerebene 36a, 38a, 40a eine Markerhalterung 116a auf. Die Markerhalterungen 116a sind zueinander abweichend ausgebildet, vorzugsweise damit diese in einem Motion Capturing eindeutig identifiziert werden können. Eine Haupterstreckungsebene der jeweiligen Markerhalterung 116a stimmt mit einer jeweiligen Trackerebene 36a, 38a, 40a überein.

Das Trackermodul 20a weist eine Trackerhalterung 32a auf. Die Markerhalterungen 116a sind gemeinsam an der einen Trackerhalterung 32a angeordnet. Die Markerhalterungen 116a sind miteinander verbunden. Alternativ könnte das Trackermodul 20a auch mehrere Trackerhalterungen 32a, vorzugsweise eine Trackerhalterung 32a für jede Trackerebene 36a, 38a, 40a und/oder Markerhalterung 116a umfassen.

Das Verfahren zum Betrieb der Endoskopiesystems 66 wird zumindest teilweise für die verschiedenen Trackerebenen 36a, 38a, 40a wiederholt. Zumindest der Kalibiriervorgang 46 wird für jede der verschiedenen Trackerebenen 36a, 38a, 40a durchgeführt. Ferner wird zumindest der Reevaluationsvorgang 50 für jede der verschiedenen Trackerebenen 36a, 38a, 40a des Trackermoduls 20a durchgeführt.

**Bezugszeichenliste**

| | | | |
|---|---|---|---|
| 10 | Endoskopbasis | 60 | Raumkamera |
| 12 | Schaft | 62 | Virtuelles Abbild |
| 14 | Handhabe | 64 | Endoskop |
| 16 | Endoskopkamera | 66 | Endoskopiesystem |
| 18 | Schnellverbinderschnittstelle | 68 | Steuereinheit |
| 20 | Trackermodul | 70 | Durchmesser |
| 22 | Speichermedium | 72 | Haupterstreckung |
| 24 | distaler Endabschnitt | 74 | Handgriff |
| 26 | Anschlag | 76 | Bedienmodul |
| 28 | Poka-Yoke-Element | 78 | Bedienelement |
| 30 | proximaler Endabschnitt | 80 | Gehäuse |
| 32 | Trackerhalterung | 82 | Beleuchtungsmodul |
| 34 | Tracker | 84 | Lichtemitter |
| 36 | Trackerebene | 86 | Bilderfassungsmodul |
| 38 | Trackerebene | 88 | Bilderfassungssensorik |
| 40 | Trackerebene | 90 | Bildsensor |
| 42 | weitere Schnellverbinderschnittstelle | 92 94 | Bilderfassungsoptik Objektiv |
| 44 | Schnellspanner | 96 | proximale Richtung |
| 46 | Kalibriervorgang | 98 | distale Richtung |
| 48 | Testvorgang | 100 | weiteres Bilderfassungsmodul |
| 50 | Reevaluationsvorgang | 102 | distales Ende |
| 52 | Bildsignal | 104 | proximales Ende |
| 54 | Reales Abbild | 106 | Ausnehmung |
| 56 | Referenzobjekt | 108 | Durchmesser |
| 58 | weiteres Bildsignal | 110 | Mantel |
| 112 | Wandung | 162 | Verfahrensschritt |
| 114 | Marker | 164 | Verfahrensschritt |
| 116 | Markerhalterung | 166 | Verfahrensschritt |
| 118 | Markerstation | 168 | Verfahrensschritt |
| 120 | Schnellverbinder | 170 | Verfahrensschritt |
| 122 | Kragenelement | 172 | Verfahrensschritt |
| 124 | Weiteres Kragenelement | 174 | Verfahrensschritt |
| 126 | Weiterer Anschlag | 176 | Verfahrensschritt |
| 128 | Weiteres Poka-Yoke-Element | | |
| 130 | Verbindungsform | | |
| 132 | Weitere Verbindungsform | | |
| 134 | Spannring | | |
| 136 | Klemm- und/oder Spannelement | | |
| 138 | Weiteres Klemm- und/oder Spannelement | | |
| 140 | Betätigungselement | | |
| 142 | elektrisches und/oder elektronisches Schnittstellenmodul | | |
| 144 | Anschluss | | |
| 146 | Energieschnittstelle | | |
| 148 | Datenschnittstelle | | |
| 150 | Rack | | |
| 152 | Steuerelektronik | | |
| 154 | Anzeige | | |
| 156 | Verfahrensschritt | | |
| 158 | Verfahrensschritt | | |
| 160 | Montagevorgang | | |

## Patentansprüche

1. Endoskopvorrichtung mit zumindest einer Endoskopbasis (10), welche wenigstens einen Schaft (12) und wenigstens eine mit dem Schaft (12) verbundene Handhabe (14) umfasst, mit wenigstens einer Endoskopkamera (16), welche zumindest teilweise in die Endoskopbasis (10) integriert ist, und mit wenigstens einer Schnellverbinderschnittstelle (18), welche zu einer lösbaren Verbindung wenigstens eines Trackermoduls (20) mit der Endoskopbasis (10) ausgebildet ist und welche wenigstens eine Lage und/oder Orientierung des Trackermoduls (20) relativ zur Endoskopkamera (16) definiert, wobei wenigstens eine werkseitig bestimmte Lage- und/oder Orientierungskenngröße dieser definierten Lage und/oder Orientierung wiederabrufbar auf wenigstens einem zugeordneten Speichermedium (22), welches zumindest teilweise in die Endoskopbasis (10) integriert ist, hinterlegt ist.

2. Endoskopvorrichtung nach Anspruch 1, **dadurch gekennzeichnet, dass** auf dem Speichermedium zumindest eine Kamerakenngröße der Endoskopkamera hinterlegt ist.

3. Endoskopvorrichtung nach Anspruch 1 oder 2, **dadurch gekennzeichnet, dass** die Endoskopkamera (16) zumindest teilweise an einem distalen Endabschnitt (24) des Schafts (12) angeordnet ist.

4. Endoskopvorrichtung nach einem der vorhergehenden Ansprüche, **dadurch gekennzeichnet, dass** die Schnellverbinderschnittstelle (18) wenigstens einen stirnseitigen Anschlag (26) umfasst, welcher dazu ausgebildet ist, die Lage des Trackermoduls (20) relativ zur Endoskopkamera (16) festzulegen.

5. Endoskopvorrichtung nach einem der vorhergehenden Ansprüche, **dadurch gekennzeichnet, dass** die Schnellverbinderschnittstelle (18) zumindest ein Poka-Yoke-Element (28) umfasst, welches dazu ausgebildet ist, die Orientierung des Trackermoduls (20) relativ zur Endoskopkamera (16) festzulegen.

6. Endoskopvorrichtung nach einem der vorhergehenden Ansprüche, **dadurch gekennzeichnet, dass** die Schnellverbinderschnittstelle (18) zumindest teilweise an einem proximalen Endabschnitt (30) des Schafts (12) angeordnet ist.

7. Endoskopvorrichtung nach einem der vorhergehenden Ansprüche, **gekennzeichnet durch** das Trackermodul (20), welches wenigstens eine Trackerhalterung (32) und wenigstens einen an der Trackerhalterung (32) angeordneten Tracker (34) umfasst.

8. Endoskopvorrichtung nach einem der vorhergehenden Ansprüche, **dadurch gekennzeichnet, dass** das Trackermodul (20) wenigstens zwei verschiedene Trackerebenen (36, 38, 40) aufweist, welche von zumindest drei Markern des Trackermoduls aufgespannt werden.

9. Endoskopvorrichtung nach einem der vorhergehenden Ansprüche, **dadurch gekennzeichnet, dass** das Trackermodul (20) wenigstens eine weitere Schnellverbinderschnittstelle (42) umfasst, welche korrespondierend zur Schnellverbinderschnittstelle (18) ausgebildet ist und mit dieser bei einer Verbindung des Trackermoduls (20) mit der Endoskopbasis (10) zusammenwirkt.

10. Endoskopvorrichtung nach Anspruch 9, **dadurch gekennzeichnet, dass** in wenigstens einer Anordnung der Schnellverbinderschnittstellen (18, 42) zueinander, die Schnellverbinderschnittstelle (18) und die weitere Schnellverbinderschnittstelle (42) stirnseitig ineinander einführbar sind und in wenigstens einer weiteren Anordnung stirnseitig aneinander anliegen.

11. Endoskopvorrichtung nach Anspruch 9 oder 10, **gekennzeichnet durch** zumindest einen Schnellspanner (44), welcher die Schnellverbinderschnittstellen (18, 42) bei einer Verbindung des Trackermoduls (20) mit der Endoskopbasis (10) durch Kraftbeaufschlagung miteinander verspannt.

12. Endoskopvorrichtung nach Anspruch 11, **dadurch gekennzeichnet, dass** der Schnellspanner (44) zumindest teilweise einstückig mit dem Trackermodul (20) ausgebildet ist.

13. Verfahren zum Betrieb einer Endoskopvorrichtung, insbesondere einer Endoskopvorrichtung nach einem der vorhergehenden Ansprüche, bei welchem in wenigstens einem Kalibriervorgang (46) wenigstens eine werkseitig bestimmte Lage- und/oder Orientierungskenngröße einer Lage und/oder Orientierung wenigstens eines Trackermoduls (20) relativ zu wenigstens einer Endoskopkamera (16), zumindest teilweise in eine Endoskopbasis (10), die wenigstens einen Schaft (12) und wenigstens eine mit dem Schaft (12) verbundene Handhabe (14) umfasst, integriert ist, wiederabrufbar auf wenigstens einem zugeordneten Speichermedium (22), welches zumindest teilweise in die Endoskopbasis (10) integriert ist, hinterlegt wird, wobei diese Lage und/oder Orientierung von wenigstens einer Schnellverbinderschnittstelle (18), welche eine lösbare Verbindung des Trackermoduls (20) mit der Endoskopbasis (10) herstellt, definiert wird.

14. Verfahren nach Anspruch 13, **dadurch gekennzeichnet, dass** in dem Kalibriervorgang (46) mittels einer werkseitigen Hand-Augen-Kalibration die Lage- und/oder Orientierungskenngröße bestimmt wird.

15. Verfahren nach Anspruch 13 oder 14, **gekennzeichnet durch** wenigstens einen Testvorgang (48), bei welchem ein Abgleich einer vorliegenden Komponentenzuordnung wenigstens des Trackermoduls (20) und der Endoskopbasis (10) mit einer auf dem Speichermedium (22) hinterlegten vorgesehenen Komponentenzuordnung durchgeführt wird.

16. Verfahren nach Anspruch 12 oder 13, **gekennzeichnet durch** zumindest einen Reevaluationsvorgang (50), bei welchem vor einem Einsatz der Endoskopvorrichtung eine auf der hinterlegten Lage- und/oder Orientierungskenngröße basierende Hand-Augen-Kalibration der Lage und/oder Orientierung des Trackermoduls (20) relativ zur Endoskopkamera (16) reevaluiert wird.

17. Verfahren nach Anspruch 16, **dadurch gekennzeichnet, dass** in dem Reevaluationsvorgang (50) ein Bildsignal (52) bereitgestellt wird, welches einem von der Endoskopkamera (16) aufgenommenen realen Abbild (54) eines Referenzobjekts (56) entspricht.

18. Verfahren nach Anspruch 16 oder 17, **dadurch gekennzeichnet, dass** in dem Reevaluationsvorgang (50) ein weiteres Bildsignal (58) bereitgestellt wird, welches einem durch erfassen des Trackermoduls (20) mittels einer separaten Raumkamera (60) und anhand der hinterlegten Lage- und/oder Ortskenngrößen erzeugten virtuellen Abbild (62) eines Referenzobjekts (56) entspricht.

19. Verfahren nach den Ansprüchen 17 und 18, **dadurch gekennzeichnet, dass** in dem Reevaluationsvorgang (50) zu einer Reevaluation der Hand-Augen-Kalibration ein Vergleich des Bildsignals (52) und des weiteren Bildsignals (58) durchgeführt wird.

20. Verfahren nach zumindest den Ansprüchen 17 und 18, **dadurch gekennzeichnet, dass** in dem Reevaluationsvorgang (50) eine Abweichung zwischen Bildsignal (52) und weiterem Bildsignal (58) bestimmt wird, welche wenigstens einer Abweichung zwischen dem reellen Abbild (54) und dem virtuellen Abbild (62) des Referenzobjekts (56) in zumindest einer Raumdimension entspricht.

21. Verfahren zumindest nach Anspruch 13 und/oder 17, **dadurch gekennzeichnet, dass** der Kalibiriervorgang (46) und/oder der Reevaluationsvorgang (50) für verschiedene Trackerebenen (36, 38, 40), welche von zumindest drei Markern des Trackermoduls aufgespannt werden, des Trackermoduls (20) durchgeführt werden.

22. Endoskop (64) mit wenigstens einer Endoskopvorrichtung nach einem der Ansprüche 1 bis 12

23. Endoskopiesystem (66) mit wenigstens einem Endoskop (64) nach Anspruch 22 und mit wenigstens einer Steuereinheit (68).

24. Endoskopiesystem (66) nach Anspruch 23, **dadurch gekennzeichnet, dass** die Steuereinheit (68) zur Durchführung des Verfahrens nach einem der Ansprüche 13 bis 21 ausgebildet ist.

## Claims

1. Endoscope device having at least one endoscope base (10) which comprises at least one shaft (12) and at least one handle (14) connected to the shaft (12), having at least one endoscope camera (16) which is at least partially integrated into the endoscope base (10), and having at least one quick connector interface (18) which is designed to releasably connect at least one tracker module (20) to the endoscope base (10) and which defines at least one position and/or orientation of the tracker module (20) relative to the endoscope camera (16), wherein at least one factory-determined position parameter and/or orientation parameter of this defined position and/or orientation is retrievably stored on at least one associated storage medium (22) which is at least partially integrated into the endoscope base (10).

2. Endoscope device according to claim 1, **characterized in that** at least one camera parameter of the endoscope camera is stored on the storage medium.

3. Endoscope device according to claim 1 or 2, **characterized in that** the endoscope camera (16) is at least partially arranged on a distal end portion (24) of the shaft (12).

4. Endoscope device according to any of the preceding claims, **characterized in that** the quick connector interface (18) comprises at least one end-face stop (26) which is designed to establish the position of the tracker module (20) relative to the endoscope camera (16).

5. Endoscope device according to any of the preceding claims, **characterized in that** the quick connector interface (18) comprises at least one poka-yoke element (28) which is designed to establish the orientation of the tracker module (20) relative to the endoscope camera (16).

6. Endoscope device according to any of the preceding claims, **characterized in that** the quick connector interface (18) is at least partially arranged on a proximal end portion (30) of the shaft (12).

7. Endoscope device according to any of the preceding claims, **characterized by** the tracker module (20) which comprises at least one tracker mount (32) and at least one tracker (34) arranged on the tracker mount (32).

8. Endoscope device according to any of the preceding claims, **characterized in that** the tracker module (20) has at least two different tracker planes (36, 38, 40) which are spanned by at least three markers of the tracker module.

9. Endoscope device according to any of the preceding claims, **characterized in that** the tracker module (20) comprises at least one further quick connector interface (42) which is designed in a manner corresponding to the quick connector interface (18) and interacts therewith when connecting the tracker module (20) to the endoscope base (10).

10. Endoscope device according to claim 9, **characterized in that** in at least one arrangement of the quick connector interfaces (18, 42) relative to each other, the quick connector interface (18) and the further quick connector interface (42) can be inserted into each other via the respective end faces thereof and in at least one further arrangement they rest against each other via the respective end faces thereof.

11. Endoscope device according to claim 9 or 10, **characterized by** at least one quick-release tensioner (44) which clamps the quick connector interfaces (18, 42) together by applying force when connecting the tracker module (20) to the endoscope base (10).

12. Endoscope device according to claim 11, **characterized in that** the quick-release tensioner (44) is at least partially formed in one piece with the tracker module (20).

13. Method for operating an endoscope device, in particular an endoscope device according to any of the preceding claims, in which method in at least one calibration process (46) at least one factory-determined position parameter and/or orientation parameter of a position and/or orientation of at least one tracker module (20) relative to at least one endoscope camera (16), at least partially integrated into an endoscope base (10) comprising at least one shaft (12) and at least one handle (14) connected to the shaft (12), is retrievably stored on at least one associated storage medium (22) which is at least partially integrated into the endoscope base (10), wherein this position and/or orientation is defined by at least one quick connector interface (18) which releasably connects the tracker module (20) to the endoscope base (10).

14. Method according to claim 13, **characterized in that** in the calibration process (46) the position parameter and/or orientation parameter is determined by means of a factory-implemented hand-eye calibration.

15. Method according to claim 13 or 14, **characterized by** at least one test process (48) in which an existing component assignment of at least the tracker module (20) and the endoscope base (10) is compared to an intended component assignment stored on the storage medium (22).

16. Method according to claim 13 or 14, **characterized by** at least one re-evaluation process (50) in which prior to use of the endoscope device a hand-eye calibration, based on the stored position parameter and/or orientation parameter, of the position and/or orientation of the tracker module (20) relative to the endoscope camera (16) is re-evaluated.

17. Method according to claim 16, **characterized in that** in the re-evaluation process (50) an image signal (52) is provided which corresponds to a real image (54) of a reference object (56) taken by the endoscope camera (16).

18. Method according to claim 16 or 17, **characterized in that** in the re-evaluation process (50) a further image signal (58) is provided which corresponds to a virtual image (62) of a reference object (56), which virtual image is generated by capturing the tracker module (20) by means of a separate room camera (60) and on the basis of the stored position parameters and/or location parameters.

19. Method according to claims 17 and 18, **characterized in that** in the re-evaluation process (50) the image signal (52) and the further image signal (58) are compared in order to re-evaluate the hand-eye calibration.

20. Method according to at least claims 17 and 18, **characterized in that** in the re-evaluation process (50) a deviation between the image signal (52) and the further image signal (58) is determined which corresponds at least to a deviation between the real image (54) and the virtual image (62) of the reference object (56) in at least one spatial dimension.

21. Method at least according to claim 13 and/or 17, **characterized in that** the calibration process (46) and/or the re-evaluation process (50) are performed for different tracker planes (36, 38, 40) of the tracker module (20) which are spanned by at least three markers of the tracker module.

22. Endoscope (64) having at least one endoscope device according to any of claims 1 to 12

23. Endoscopy system (66) having at least one endoscope (64) according to claim 22 and at least one control unit (68).

24. Endoscopy system (66) according to claim 23, **characterized in that** the control unit (68) is designed to perform the method according to any of claims 13 to 21.

## Revendications

1. Dispositif endoscopique comportant au moins une base d'endoscope (10) qui comprend au moins une tige (12) et au moins une poignée (14) reliée à la tige (12), comportant au moins une caméra d'endoscope (16) qui est au moins partiellement intégrée dans la base d'endoscope (10), et comportant au moins une interface de liaison rapide (18) qui est conçue pour une liaison amovible d'au moins un module à suiveur (20) avec la base d'endoscope (10) et qui définit au moins une position et/ou une orientation du module à suiveur (20) par rapport à la caméra d'endoscope (16), dans lequel au moins un paramètre de position et/ou d'orientation déterminé en usine de ladite position et/ou orientation définies est enregistré de manière à pouvoir être rappelé sur au moins un support de mémorisation (22) associé qui est intégré au moins partiellement dans la base d'endoscope (10).

2. Dispositif endoscopique selon la revendication 1, **caractérisé en ce qu'**au moins un paramètre de caméra de la caméra d'endoscope est enregistré sur le support de mémorisation.

3. Dispositif endoscopique selon la revendication 1 ou 2, **caractérisé en ce que** la caméra d'endoscope (16) est disposée au moins partiellement sur une section d'extrémité distale (24) de la tige (12).

4. Dispositif endoscopique selon l'une des revendications précédentes, **caractérisé en ce que** l'interface de liaison rapide (18) comprend au moins une butée frontale (26) conçue pour fixer la position du module à suiveur (20) par rapport à la caméra d'endoscope (16).

5. Dispositif endoscopique selon l'une des revendications précédentes, **caractérisé en ce que** l'interface de liaison rapide (18) comprend au moins un élément de type Poka-Yoke (28) conçu pour fixer l'orientation du module à suiveur (20) par rapport à la caméra d'endoscope (16).

6. Dispositif endoscopique selon l'une des revendications précédentes, **caractérisé en ce que** l'interface de liaison rapide (18) est disposée au moins partiellement sur une section d'extrémité proximale (30) de la tige (12).

7. Dispositif endoscopique selon l'une des revendications précédentes, **caractérisé par** le module à suiveur (20) qui comprend au moins un moyen de maintien de suiveur (32) et au moins un suiveur (34) disposé sur le moyen de maintien de suiveur (32).

8. Dispositif endoscopique selon l'une des revendications précédentes, **caractérisé en ce que** le module à suiveur (20) présente au moins deux plans de suiveur (36, 38, 40) différents qui sont définis par au moins trois marqueurs du module à suiveur.

9. Dispositif endoscopique selon l'une des revendications précédentes, **caractérisé en ce que** le module à suiveur (20) comprend au moins une autre interface de liaison rapide (42) qui est conçue de manière à correspondre à l'interface de liaison rapide (18) et qui coopère avec celle-ci lors d'une liaison du module à suiveur (20) avec la base d'endoscope (10).

10. Dispositif endoscopique selon la revendication 9, **caractérisé en ce que,** dans au moins un agencement des interfaces de liaison rapide (18, 42) l'une par rapport à l'autre, l'interface de liaison rapide (18) et l'autre interface de liaison rapide (42) peuvent être insérées l'une dans l'autre côté frontal et s'appuient l'une contre l'autre côté frontal dans au moins un autre agencement.

11. Dispositif endoscopique selon la revendication 9 ou 10, **caractérisé par** au moins un moyen de serrage rapide (44) qui serre les interfaces de liaison rapide (18, 42) l'une contre l'autre par application d'une force lors d'une liaison du module à suiveur (20) à la base d'endoscope (10).

12. Dispositif endoscopique selon la revendication 11, **caractérisé en ce que** le moyen de serrage rapide (44) est formé au moins partiellement d'une seule pièce avec le module à suiveur (20).

13. Procédé permettant de faire fonctionner un dispositif endoscopique, en particulier un dispositif endoscopique selon l'une des revendications précédentes, dans lequel, lors d'au moins un processus de calibrage (46), au moins un paramètre de position et/ou d'orientation déterminé en usine d'une position et/ou d'une orientation d'au moins un module à suiveur (20) par rapport à au moins une caméra d'endoscope (16), est intégrée au moins partiellement dans une base d'endoscope (10) qui comprend au moins une tige (12) et au moins une poignée (14) reliée à la tige (12), est enregistré de manière à pouvoir être rappelé sur au moins un support de mémorisation (22) associé qui est au moins partiellement intégré dans la base d'endoscope (10), dans lequel ladite position et/ou orientation sont définies par au moins une interface de liaison rapide (18) qui établit une liaison amovible du module à suiveur (20) avec la base d'endoscope (10).

14. Procédé selon la revendication 13, **caractérisé en ce que,** lors du processus de calibrage (46), le paramètre de position et/ou d'orientation est déterminé au moyen d'un calibrage manuel à l'œil réalisé en usine.

15. Procédé selon la revendication 13 ou 14, **caractérisé par** au moins un processus de test (48), lors duquel une comparaison d'une association de composants présente d'au moins le module à suiveur (20) et la base d'endoscope (10) avec une association de composants prévue enregistrée sur le support de mémorisation (22) est réalisée.

16. Procédé selon la revendication 12 ou 13, **caractérisé par** au moins un processus de réévaluation (50), lors duquel, avant une utilisation du dispositif endoscopique, une calibration main-œil de la position et/ou de l'orientation du module à suiveur (20) par rapport à la caméra d'endoscope (16), laquelle calibration est basée sur le paramètre de position et/ou d'orientation enregistré, est réévaluée.

17. Procédé selon la revendication 16, **caractérisé en ce que,** lors du processus de réévaluation (50), un signal d'image (52) est fourni, lequel correspond à une image réelle (54) d'un objet de référence (56) prise par la caméra d'endoscope (16).

18. Procédé selon la revendication 16 ou 17, **caractérisé en ce que,** lors du processus de réévaluation (50), un autre signal d'image (58) est fourni, lequel correspond à une image virtuelle (62) d'un objet de référence (56) générée par la détection du module à suiveur (20) au moyen d'une caméra spatiale (60) séparée et à l'aide des paramètres de position et/ou d'emplacement enregistrés.

19. Procédé selon les revendications 17 et 18, **caractérisé en ce que,** lors du processus de réévaluation (50), une comparaison du signal d'image (52) et de l'autre signal d'image (58) est réalisée pour une réévaluation de la calibration main-œil.

20. Procédé selon au moins les revendications 17 et 18, **caractérisé en ce que,** lors du processus de réévaluation (50), un écart entre le signal d'image (52) et un autre signal d'image (58) est déterminé, lequel écart correspond au moins à un écart entre l'image réelle (54) et l'image virtuelle (62) de l'objet de référence (56) dans au moins une dimension spatiale.

21. Procédé au moins selon la revendication 13 et/ou 17, **caractérisé en ce que** le processus de calibrage (46) et/ou le processus de réévaluation (50) sont réalisés pour différents plans de suiveur (36, 38, 40) du module à suiveur (20) qui sont définis par au moins trois marqueurs du module à suiveur.

22. Endoscope (64) comportant au moins un dispositif endoscopique selon l'une des revendications 1 à 12.

23. Système endoscopique (66) comportant au moins un endoscope (64) selon la revendication 22 et comportant au moins une unité de commande (68).

24. Système endoscopique (66) selon la revendication 23, **caractérisé en ce que** l'unité de commande (68) est configurée pour réaliser le procédé selon l'une des revendications 13 à 21.
